# EUROPEAN PATENT SPECIFICATION

(11) **EP 3 900 700 B1**
(45) Date of publication and mention of the grant of the patent: **13.09.2023**
(21) Application number: 19900452.4
(22) Date of filing: 11.11.2019
(51) Int. Cl.: A61K 8/895, A61K 8/06, A61K 8/34, A61K 8/64, A61K 8/891, A61Q 1/00, A61Q 17/04, A61Q 19/00

(54) **MICROEMULSION COMPOSITION, CURED PRODUCT THEREOF, AND COSMETIC MATERIAL CONTAINING SAME**
MIKROEMULSIONSZUSAMMENSETZUNG, GEHÄRTETES PRODUKT DARAUS UND KOSMETISCHES MATERIAL DAMIT
COMPOSITION DE MICROÉMULSION, PRODUIT DURCI DE CELLE-CI, ET MATÉRIAU COSMÉTIQUE LE CONTENANT

(30) Priority: 19.12.2018 JP 2018237526
(43) Date of publication of application: 27.10.2021
(73) Proprietor: Shin-Etsu Chemical Co., Ltd., Tokyo 100-0005 (JP)
(72) Inventor: ABE, Takuya, Annaka-shi, Gunma 379-0224 (JP)
(74) Representative: Wibbelmann, Jobst
(86) International application number: PCT/JP2019/044045
(87) International publication number: WO 2020/129461

(56) References cited:
- WO-A1-2012/165227
- WO-A1-2017/160349
- WO-A1-2018/008653
- WO-A1-2018/218417
- JP-A- H1 171 459
- JP-B2- 2 575 737

## Description

### TECHNICAL FIELD

The present invention relates to: a microemulsion composition; a cured material thereof; and a cosmetic containing the cured material.

### BACKGROUND ART

Transparent or translucent microemulsions containing a surfactant, an aqueous phase, and an oil phase are broadly divided into three types: a water-dispersion emulsion, whose continuous phase is water; an oil-dispersion emulsion, whose continuous phase is oil; and a bicontinuous emulsion, whose continuous phase is formed from water and oil. In particular, a microemulsion composition having a bicontinuous structure can achieve a cosmetic with improved functionality and feeling on use, and examples include compositions used for cleansing agents or cleaning agents of skin or hair (Patent Documents 1 to 7).

Bicontinuous microemulsion compositions require the use of a large amount of surfactant compared with water-dispersion emulsions or oil-dispersion emulsions. However, since stickiness or an oily feeling originating from an activator remain when used as a cosmetic, it is necessary to reduce the amount of surfactant used in order to maintain light feeling (Patent Document 8). In addition, when a silicone oil is used for the oil phase, feeling can be improved, but there are few reported examples, and the type of surfactant becomes limited.

A microemulsion composition containing a silicone oil can be produced by using Surfactin, which is a natural surfactant. Patent Document 9 reports an emulsified composition containing Surfactin, amino-modified silicone, and an aqueous solvent. The emulsified composition is characteristic in that the emulsified composition has a high emulsifying capacity, and a small amount of surfactant suffices. However, since the activator is anionic, a strong feeling of stickiness originating from the activator remains compared with a nonionic surfactant. In addition, usable silicones are limited, and there are no reported examples using an organopolysiloxane having a reactive functional group such as a hydrosilyl group or an olefinic unsaturated group.

Meanwhile, as a means to reduce stickiness, the use of silicone particles that can provide dry or smooth feeling on use and spreadability are known (Patent Documents 10 and 11). In particular, fine silicone particles formed by coating silicone rubber spherical particles with polyorganosilsesquioxane have soft feeling, and is excellent in dispersibility, and are therefore blended in many cosmetics. However, production methods thereof all go through a water-dispersion emulsion with a clouded appearance, and there is no report disclosed of going through a microemulsion with a transparent appearance.

### CITATION LIST

### PATENT LITERATURE

Patent Document 1 JP 2009-196909 A
Patent Document 2 JP 2015-105255 A
Patent Document 3 JP 2017-66085 A
Patent Document 4 JP 2004-217640 A
Patent Document 5 JP 2013-32348 A
Patent Document 6 JP 2014-224061 A
Patent Document 7 JP 2010-222324 A
Patent Document 8 JP 2011-178769 A
Patent Document 9 WO 2018/008653
Patent Document 10 JP 2010-132877 A
Patent Document 11 JP 2017-193702 A
Patent Document 12 WO 2015/022936

### SUMMARY OF INVENTION

### TECHNICAL PROBLEM

The present invention has been made in view of the above-described circumstances, and an object thereof is: to provide a microemulsion composition having a transparent or translucent appearance when an organopolysiloxane having reactive functional groups such as hydrosilyl groups and olefinic unsaturated groups is used for an oil phase; to provide a microemulsion addition-cured composition while maintaining transparency; and to provide a cosmetic containing the microemulsion addition-cured composition.

### SOLUTION TO PROBLEM

To achieve the above-described object, the present invention provides
a microemulsion composition comprising:
(A) an anionic surfactant;
(B) an organopolysiloxane having at least two hydrosilyl groups in one molecule thereof shown in the following formula (I) : 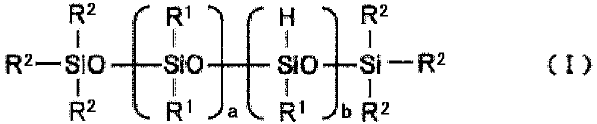 wherein each R¹ independently represents a substituted or unsubstituted monovalent hydrocarbon group having 1 to 30 carbon atoms and not having an aliphatic unsaturated bond, each R² independently represents a substituted or unsubstituted monovalent hydrocarbon group having 1 to 30 carbon atoms and not having an aliphatic unsaturated bond, some optionally being a hydrogen atom, "a" satisfies 0 ≤ a ≤ 300, "b" satisfies 0 ≤ b ≤ 50, and 5 ≤ a + b ≤ 350, and when b = 0, any two or more R² represent a hydrogen atom;
(C) an organopolysiloxane having at least two olefinic unsaturated groups in one molecule thereof shown in the following formula (II): 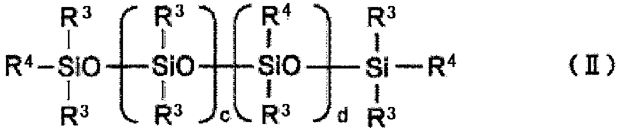 wherein each R³ independently represents a substituted or unsubstituted monovalent hydrocarbon group having 1 to 30 carbon atoms and not having an aliphatic unsaturated bond, each R⁴ independently represents a substituted or unsubstituted monovalent hydrocarbon group having 2 to 30 carbon atoms and having an aliphatic unsaturated bond or is R³, "c" satisfies 0 ≤ c ≤ 500, "d" satisfies 0 ≤ d ≤ 50, and 5 ≤ c + d ≤ 550, and when d = 0, each R⁴ independently represents a substituted or unsubstituted monovalent hydrocarbon group having 2 to 30 carbon atoms and having an aliphatic unsaturated bond;
(D) a monohydric or polyhydric alcohol; and
(E) water,
wherein the microemulsion composition has a transparent or translucent appearance at 25°C.

The inventive microemulsion composition allows, by addition of a hydrosilylation catalyst, an addition-curing reaction without losing a transparent appearance, and a transparent or translucent microemulsion addition-cured composition can be produced.

In this event, the microemulsion composition is preferably dispersible when added in water.

Such a microemulsion composition can be used suitably for cosmetics, etc.

Furthermore, the (A) anionic surfactant is preferably a natural surfactant.

Such an (A) anionic surfactant can reduce load on the environment, is highly safe, and can be used suitably in a microemulsion composition.

Furthermore, the natural surfactant preferably comprises a cyclic peptide group shown in the following formula (III) : 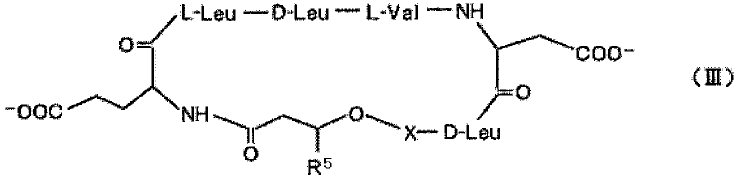 wherein in the formula, X represents an amino acid residue selected from leucine, isoleucine, and valine, each R⁵ independently represents a substituted or unsubstituted monovalent hydrocarbon group having 9 to 18 carbon atoms and not having an aliphatic unsaturated bond, L-Leu indicates L-leucine, D-Leu indicates D-leucine, L-Val indicates L-valine, and a counter ion of a carboxy group comprises an alkali metal ion.

When the natural surfactant is a natural surfactant that can be shown by the formula (III) as described, the natural surfactant can be used more suitably in a microemulsion composition.

In this case, in the formula (III), X preferably represents leucine, and R³ preferably represents a hydrocarbon chain having 12 carbon atoms.

When the natural surfactant shown in the formula (III) is as described, the natural surfactant can be used further suitably in a microemulsion composition.

Furthermore, the (A) anionic surfactant in the microemulsion composition is preferably contained in an amount of 0.1 to 10 wt%.

When the (A) anionic surfactant is contained in such an amount, sufficient microemulsion can be formed.

Furthermore, the (D) monohydric or polyhydric alcohol is preferably glycerin.

When the (D) monohydric or polyhydric alcohol is glycerin, a D phase (surfactant phase) can be formed in a wide range of concentrations.

Furthermore, the microemulsion composition preferably forms a bicontinuous structure.

When the microemulsion composition is a microemulsion composition that forms a bicontinuous structure as described, functionality and feeling on use can be improved when a cosmetic is prepared using the microemulsion composition.

Furthermore, there is preferably 0.5 to 3.0 mol of the hydrosilyl groups contained in the (B) relative to 1 mol of the olefinic unsaturated groups contained in the (C) .

When the hydrosilyl groups contained in the (B) is contained in such a molar quantity, an addition-curing reaction progresses sufficiently when the microemulsion composition is addition-cured, and sufficient feeling can be achieved. In addition, transparency after addition-curing can be maintained.

Furthermore, the microemulsion composition is preferably addition-cured by adding (F) a hydrosilylation catalyst.

Thus, the microemulsion composition can be addition-cured by adding (F) a hydrosilylation catalyst.

Furthermore, the present invention provides a microemulsion addition-cured composition obtained by addition-curing the inventive microemulsion composition, wherein the microemulsion addition-cured composition has a transparent or translucent appearance.

Such a microemulsion addition-cured composition has a transparent appearance, and has reduced stickiness originating from an anionic surfactant.

Furthermore, the present invention provides a cosmetic comprising the above-described microemulsion addition-cured composition.

A cosmetic having the above-described microemulsion addition-cured composition blended as described has reduced stickiness originating from an anionic surfactant, a favorable feeling on use, refreshing feeling, and temporal stability, favorable cosmetic sustainability, favorable smooth spreadability and finish, and excellent abrasion resistance.

### ADVANTAGEOUS EFFECTS OF INVENTION

The inventive microemulsion composition allows, by addition of a hydrosilylation catalyst, an addition-curing reaction without losing a transparent appearance, and a transparent or translucent microemulsion addition-cured composition can be produced. A cosmetic having the microemulsion addition-cured composition blended has reduced stickiness originating from an anionic surfactant, a favorable feeling on use, refreshing feeling, and temporal stability, favorable cosmetic sustainability, favorable smooth spreadability and finish, and excellent abrasion resistance.

### DESCRIPTION OF EMBODIMENTS

Hereinafter, the present invention will be described in detail. However, the present invention is not limited thereto.

To achieve the above object, the present inventor has earnestly studied and found out that when organopolysiloxanes shown by the following formulae (I) and (II) having reactive functional groups such as hydrosilyl groups and olefinic unsaturated groups are an oil phase, a microemulsion composition having a transparent or translucent appearance can be easily provided. Furthermore, the present inventor has found out that by adding a hydrosilylation catalyst to the microemulsion composition, a transparent or translucent microemulsion addition-cured composition can be produced. The present inventor has further found out that a cosmetic containing the obtained microemulsion addition-cured composition can achieve reduced stickiness originating from an activator, and an excellent feeling on use, refreshing feeling, and temporal stability, and arrived at the present invention.

That is, the present invention is a microemulsion composition containing the following (A) to (E), the microemulsion composition having a transparent or translucent appearance at 25°C.
(A) An anionic surfactant
(B) An organopolysiloxane having at least two hydrosilyl groups in one molecule thereof shown in the following formula (I) : 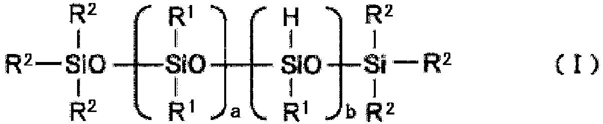 wherein each R¹ independently represents a substituted or unsubstituted monovalent hydrocarbon group having 1 to 30 carbon atoms and not having an aliphatic unsaturated bond, each R² independently represents a substituted or unsubstituted monovalent hydrocarbon group having 1 to 30 carbon atoms and not having an aliphatic unsaturated bond, some optionally being a hydrogen atom, "a" satisfies 0 ≤ a ≤ 300, "b" satisfies 0 ≤ b ≤ 50, and 5 ≤ a + b ≤ 350, and when b = 0, any two or more R² represent a hydrogen atom
(C) An organopolysiloxane having at least two olefinic unsaturated groups in one molecule thereof shown in the following formula (II): 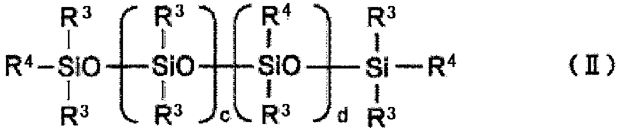 wherein each R³ independently represents a substituted or unsubstituted monovalent hydrocarbon group having 1 to 30 carbon atoms and not having an aliphatic unsaturated bond, each R⁴ independently represents a substituted or unsubstituted monovalent hydrocarbon group having 2 to 30 carbon atoms and having an aliphatic unsaturated bond or is R³, "c" satisfies 0 ≤ c ≤ 500, "d" satisfies 0 ≤ d ≤ 50, and 5 ≤ c + d ≤ 550, and when d = 0, each R⁴ independently represents a substituted or unsubstituted monovalent hydrocarbon group having 2 to 30 carbon atoms and having an aliphatic unsaturated bond
(D) A monohydric or polyhydric alcohol
(E) Water

Such a microemulsion composition allows, by addition of a hydrosilylation catalyst, an addition-curing reaction without losing a transparent appearance, and a transparent or translucent microemulsion addition-cured composition can be produced.

### [Component A]

The (A) anionic surfactant used in the present invention has an anionic hydrophilic group in the molecular structure thereof, and has a hydrophobic group including a linear or branched hydrocarbon chain, an aromatic ring, or a heterocycle, and a composite thereof. Examples include a fatty acid soap such as sodium stearate and triethanolamine palmitate, an alkyl ether carboxylic acid and a salt thereof, a condensate between an amino acid and a fatty acid, an alkane sulfonate, an alkene sulfonate, a sulfonate of a fatty acid ester, a sulfonate of a fatty acid amide, a sulfonate of a formalin condensate, an alkyl sulfate ester salt, a sulfate ester salt of a secondary alcohol, a sulfate ester salt of an alkyl and an allyl ether, a sulfate ester salt of a fatty acid ester, a sulfate ester salt of a fatty acid alkylolamide, a sulfate ester salt of a Turkey red oil and so on, an alkyl phosphate salt, an ether phosphate salt, an alkyl allyl ether phosphate salt, an amide phosphate salt, an N-acyl lactate, an N-acyl sarcosinate, an N-acylamino acid activator, and natural surfactants exemplified by lecithin, bile acid, and Surfactin. One of these can be used or a combination of two or more thereof can be used. In particular, a natural surfactant is preferably used.

A natural surfactant is a surfactant derived from a biological component that is said to be highly safe, since a natural surfactant reduces load on the environment. Natural surfactants have a peculiar chemical structure in that natural surfactants are bulky and have polyfunctionality compared with common synthetic surfactants, and do not contain components derived from petroleum. Therefore, natural surfactants have characteristics such as special functionality, biodegradability, low toxicity, and bioactivity, and are attracting attention in recent years. Specific examples include lecithin, bile acid, and Surfactin, etc., and among these natural surfactants, Surfactin is particularly favorably used.

Surfactin is a natural surfactant containing a cyclic peptide group shown in the following formula (III). Surfactin is a biosurfactant produced from Bacillus subtilis, and has a hydrophilic part having a cyclic peptide structure with seven amino acids as constituents and a hydrophobic part including a hydrocarbon group. Surfactin is a general term for compounds with hydrocarbon groups of different alkyl chain lengths and branching degree. Surfactin Na, being a sodium salt, has low skin irritation compared with other anionic surfactants. In addition, Surfactin Na has a characteristic that the critical micelle concentration shows an extremely low value of 0.0003 wt% by cyclic peptide structures attracting each other between molecules by hydrogen bonds. 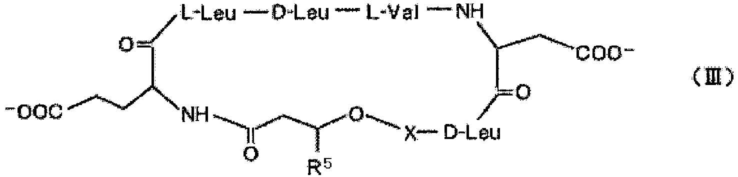 In the formula, X represents an amino acid residue selected from leucine, isoleucine, and valine, each R⁵ independently represents a substituted or unsubstituted monovalent hydrocarbon group having 9 to 18 carbon atoms and not having an aliphatic unsaturated bond, L-Leu indicates L-leucine, D-Leu indicates D-leucine, L-Val indicates L-valine, and a counter ion of a carboxy group includes an alkali metal ion.

X represents an amino acid residue selected from leucine, isoleucine, and valine, preferably leucine.

Each R⁵ independently represents a substituted or unsubstituted monovalent hydrocarbon group having 9 to 18 carbon atoms and not having an aliphatic unsaturated bond. Examples include alkyl groups, aryl groups, and aralkyl groups having 9 to 18 carbon atoms. More specific examples include a nonyl group, a decyl group, an undecyl group, a dodecyl group, a tridecyl group, a tetradecyl group, and the like. In particular, a nonyl group, a decyl group, an undecyl group, and a dodecyl group, having 9 to 12 carbon atoms, are preferable.

In the natural surfactant (Surfactin) containing the cyclic peptide group shown in the formula (III), the amino acid residue is preferably leucine and R⁵ preferably represents a branched hydrocarbon chain having 12 carbon atoms, and more preferably, a counter ion of the carboxy group is a sodium ion. Those disclosed in Patent Document 12 can be used, for example. Such substances are not limited to the following example, but "KANEKA Surfactin" manufactured by Kaneka Corporation can be used, for example.

### [Component B]

The (B) organopolysiloxane having at least two hydrosilyl groups in one molecule thereof used in the present invention is shown by the following formula (I): 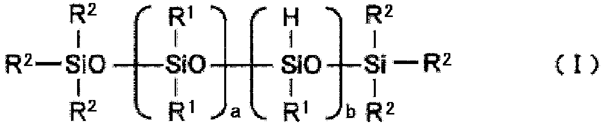 where each R¹ independently represents a substituted or unsubstituted monovalent hydrocarbon group having 1 to 30 carbon atoms and not having an aliphatic unsaturated bond, each R² independently represents a substituted or unsubstituted monovalent hydrocarbon group having 1 to 30 carbon atoms and not having an aliphatic unsaturated bond, some optionally being a hydrogen atom, "a" satisfies 0 ≤ a ≤ 300, "b" satisfies 0 ≤ b ≤ 50, and 5 ≤ a + b ≤ 350, and when b = 0, any two or more R² represent a hydrogen atom, and when b = 1, any one or more R² represent a hydrogen atom.

In the formula (I), each R¹ independently represents a substituted or unsubstituted monovalent hydrocarbon group having 1 to 30 carbon atoms, preferably 1 to 10 carbon atoms and not having an aliphatic unsaturated bond. Examples include alkyl groups, aryl groups, and aralkyl groups having 1 to 30 carbon atoms, or a group obtained by substituting a hydrogen atom bonded to a carbon atom of these groups with a halogen atom, an amino group, or a carboxy group. In particular, alkyl groups, aryl groups, and aralkyl groups having 1 to 10 carbon atoms, fluorine-substituted alkyl groups, chloro-substituted alkyl groups, amino-substituted alkyl groups, and carboxyl-substituted alkyl groups are preferable. More specific examples include a methyl group, an ethyl group, a propyl group, a butyl group, a pentyl group, a cyclopentyl group, a cyclohexyl group, a phenyl group, a tolyl group, a trifluoropropyl group, a heptadecafluorodecyl group, a chloropropyl group, a chlorophenyl group, and the like. In particular, an alkyl group having 1 to 5 carbon atoms, a phenyl group, or a trifluoropropyl group is preferable.

In the formula (I), each R² independently represents a substituted or unsubstituted monovalent hydrocarbon group having 1 to 30 carbon atoms, preferably 1 to 10 carbon atoms and not having an aliphatic unsaturated bond, or represents a hydrogen atom. Examples of the hydrocarbon group include alkyl groups, aryl groups, and aralkyl groups having 1 to 30 carbon atoms, or a group obtained by substituting a hydrogen atom bonded to a carbon atom of these groups with a halogen atom, an amino group, or a carboxy group. In particular, alkyl groups, aryl groups, and aralkyl groups having 1 to 10 carbon atoms, fluorine-substituted alkyl groups, chloro-substituted alkyl groups, amino-substituted alkyl groups, and carboxyl-substituted alkyl groups are preferable. More specific examples include a methyl group, an ethyl group, a propyl group, a butyl group, a pentyl group, a cyclopentyl group, a cyclohexyl group, a phenyl group, a tolyl group, a trifluoropropyl group, a heptadecafluorodecyl group, a chloropropyl group, a chlorophenyl group, and the like. In particular, an alkyl group having 1 to 5 carbon atoms, a phenyl group, a trifluoropropyl group, or a hydrogen atom is preferable.

In the formula (I), "a" satisfies 0 ≤ a ≤ 300, preferably 10 ≤ a ≤ 100. If "a" is greater than 300, the molecular structure becomes large, so that stability after emulsification becomes poor. "b" satisfies 0 ≤ b ≤ 50, preferably 0 ≤ b ≤ 30. If "b" is greater than 50, the number of crosslinking points in addition reaction becomes large, and therefore, the obtained cured material becomes hard so that feeling becomes poor. a + b satisfies 5 ≤ a + b ≤ 350, preferably 10 ≤ a + b ≤ 150. If a + b is less than 5, the molecular weight of the cured material after the addition reaction becomes low, so that the quality of the obtained cured material becomes close to liquid, and feeling becomes poor. If a + b is greater than 350, the molecular structure becomes large, and therefore, stability after emulsification becomes poor.

### [Component C]

The (C) organopolysiloxane having at least two olefinic unsaturated groups in one molecule thereof used in the present invention is shown in the following formula (II): 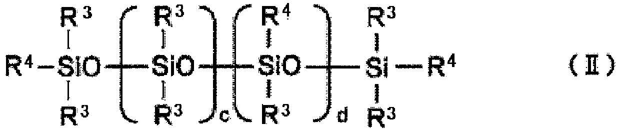 where each R³ independently represents a substituted or unsubstituted monovalent hydrocarbon group having 1 to 30 carbon atoms and not having an aliphatic unsaturated bond, each R⁴ independently represents a substituted or unsubstituted monovalent hydrocarbon group having 2 to 30 carbon atoms and having an aliphatic unsaturated bond or is R³, "c" satisfies 0 ≤ c ≤ 500, "d" satisfies 0 ≤ d ≤ 50, and 5 ≤ c + d ≤ 550, and when d = 0, each R⁴ independently represents a substituted or unsubstituted monovalent hydrocarbon group having 2 to 30 carbon atoms and having an aliphatic unsaturated bond.

In the formula (II), each R³ independently represents a substituted or unsubstituted monovalent hydrocarbon group having 1 to 30 carbon atoms, preferably 1 to 10 carbon atoms and not having an aliphatic unsaturated bond. Examples include alkyl groups, aryl groups, and aralkyl groups having 1 to 30 carbon atoms, or a group obtained by substituting a hydrogen atom bonded to a carbon atom of these groups with a halogen atom, an amino group, or a carboxy group. In particular, alkyl groups, aryl groups, and aralkyl groups having 1 to 10 carbon atoms, fluorine-substituted alkyl groups, chloro-substituted alkyl groups, amino-substituted alkyl groups, and carboxyl-substituted alkyl groups are preferable. More specific examples include a methyl group, an ethyl group, a propyl group, a butyl group, a pentyl group, a cyclopentyl group, a cyclohexyl group, a phenyl group, a tolyl group, a trifluoropropyl group, a heptadecafluorodecyl group, a chloropropyl group, a chlorophenyl group, and the like. In particular, an alkyl group having 1 to 5 carbon atoms, a phenyl group, or a trifluoropropyl group is preferable.

Furthermore, in the formula (II), each R⁴ independently represents a substituted or unsubstituted monovalent hydrocarbon group having 2 to 30 carbon atoms, preferably 2 to 10 carbon atoms having an aliphatic unsaturated bond or is a similar group to R³. Examples of the hydrocarbon group having an aliphatic unsaturated bond include a vinyl group, an allyl group, a propenyl group, a hexenyl group, a styryl group, and the like. In particular, a vinyl group is preferable. When R⁴ is the same as R³, R⁴ is as described above.

In the formula (II), "c" satisfies 0 ≤ c ≤ 500, and "d" satisfies 0 ≤ d ≤ 50. "c" satisfies 0 ≤ c ≤ 500, preferably 10 ≤ c ≤ 400. If "c" is greater than 500, the molecular structure becomes large, so that stability after emulsification becomes poor. "d" satisfies 0 ≤ d ≤ 50, preferably 0 ≤ d ≤ 30, further preferably 0 ≤ d ≤ 10. If "d" is greater than 50, the number of crosslinking points in addition reaction becomes large, and therefore, the obtained cured material becomes hard so that feeling becomes poor. c + d satisfies 5 ≤ c + d ≤ 550, preferably 10 ≤ c + d ≤ 400. If c + d is less than 5, the molecular weight of the cured material after the addition reaction becomes low, so that the quality of the obtained cured material becomes close to liquid, and feeling becomes poor. If c + d is greater than 550, the molecular structure becomes large, and therefore, stability after emulsification becomes poor.

### [Component D]

Examples of the (D) monohydric or polyhydric alcohol used in the present invention include generally used monohydric alcohols and polyhydric alcohols. Specific examples include a lower or higher, primary alcohol, sugar alcohols such as erythritol, maltitol, xylitol, and sorbitol, and polyhydric alcohols such as 1,3-BG, glycerin, PG, and DPG. One of these can be used or an appropriate combination of two or more thereof can be used. In particular, a water-soluble polyhydric alcohol is preferably used.

As the polyhydric alcohol, one of 1,2-alkanediol having 5 to 10 carbon atoms and a polyhydric alcohol other than 1,2-alkanediol having 5 to 10 carbon atoms or a combination of two or more thereof is preferably used. When a polyhydric alcohol is used, HLB (Hydrophilic-Liphilic Balance) can be adjusted by combining with (A) an anionic surfactant, and a D phase can be easily formed.

Specific examples of the 1,2-alkanediol having 5 to 10 carbon atoms include 1,2-pentanediol, 1,2-hexanediol, 1,2-heptanediol, 1,2-octanediol, 1,2-nonanediol, and 1,2-decanediol. In particular, one or more out of 1,2-hexanediol, 1,2-heptanediol, and 1,2-octanediol is preferably used.

The polyhydric alcohol other than 1,2-alkanediol having 5 to 10 carbon atoms is not particularly limited as long as the polyhydric alcohol is used as raw material for cosmetics. Examples thereof include ethylene glycol, diethylene glycol, polyethylene glycol, propylene glycol, dipropylene glycol, polypropylene glycol, glycerin, diglycerin, polyglycerin, 1,3-butyleneglycol, isoprene glycol, sorbitol, mannitol, and glycol. In particular, dipropylene glycol, glycerin, and 1,3-butyleneglycol are preferable. Furthermore, glycerin is particularly preferable since a D phase can be formed in a wide range of concentrations when glycerin is used.

The total amount of the (D) monohydric or polyhydric alcohol blended is preferably 1.0 to 70 mass% of the cosmetic, more preferably 5 to 50 mass%. When the blended amount is 1.0 mass% or more, sufficient microemulsion can be obtained.

### [Method for Manufacturing Microemulsion]

A common emulsifying and dispersing apparatus can be used for emulsification, and examples thereof include high-speed rotary centrifugal stirrers such as a homogenizing disper, high-speed rotary shear stirrers such as a homomixer, high-pressure jetting emulsifying and dispersing apparatuses such as a homogenizer, a colloid mill, an ultrasonic emulsifier, and the like.

When mixing the five components (A) to (E), a phase inversion temperature emulsification method, a D phase emulsification method, or the like can be employed (emulsification step).

A phase inversion temperature emulsification method is a method of stirring near a phase inversion temperature (PIT) at which the HLB becomes balanced, and then cooling quickly to produce a fine emulsion. Near a PIT, the surface tension between oil and water becomes remarkably low, and therefore, fine emulsified particles are easily produced.

In a D phase emulsification method, water-soluble polyhydric alcohol can be added to adjust the HLB of the surfactant and form a D phase, then oil can be added to go through an O/D emulsion and water can be added to produce a fine emulsion.

In the D phase emulsification method, specifically, the organopolysiloxanes of component (B) and component (C) are blended gradually under conditions of shearing with a homogenizing disper into a mixture of (A) an anionic surfactant and a polyhydric alcohol as component (D) to form a D phase. A transparent or translucent microemulsion can be obtained by subsequently adding a predetermined amount of (E) water gradually.

There are three types of microemulsions: an aqueous micelle solution phase, where oil is made soluble in water; a reverse micelle oil solution phase, where water is made soluble in oil; and a bicontinuous phase, where both water and oil take on a continuous structure, and a microemulsion falls under one of these phases.

Whether a microemulsion composition is aqueous or oily can be determined by the following method. When several drops of the microemulsion composition are rapidly dispersed homogeneously to excess water after being dropped thereto and are not dispersed to excess oil after being dropped thereto, it is aqueous. On the other hand, when they are rapidly dispersed homogeneously to excess oil after being dropped thereto and are not dispersed to excess water after being dropped thereto, it is oily. An aqueous micelle solution, where oil is made soluble in water, is aqueous since the aqueous micelle solution is rapidly dispersed when added in water, and is not dispersed when added in oil. In the case of a reverse micelle oil solution phase, where water is made soluble in oil, the reverse micelle oil solution phase is oily since it is not dispersed when added in water, and is rapidly dispersed when added in oil. A bicontinuous phase, where both water and oil take on a continuous structure, is either aqueous or oily. The inventive microemulsion composition is preferably dispersed when added in water. That is, the inventive microemulsion composition is preferably either an aqueous micelle solution, where oil is made soluble in water, or a bicontinuous phase, where both water and oil take on a continuous structure.

Furthermore, whether a microemulsion composition is an aqueous micelle solution phase or a bicontinuous phase can be determined by the following method.

The inventive microemulsion composition preferably forms a bicontinuous structure. It can be confirmed that the composition has a bicontinuous structure by observing an electron microscope image using a freeze-fracture replica technique of known method. More conveniently, it can be confirmed by a solubility test of pigments. The solubility test of pigments is a method of adding each of aqueous pigments and oily pigments to confirm that the composition is amphiphilic when it is rapidly mixed with both of water and oil.

In the inventive microemulsion composition, 0.1 to 10 wt% of the (A) anionic surfactant is preferably contained. An emulsion can be formed with a small amount added, since the surfactant is excellent in emulsification performance.

In the inventive microemulsion composition, the content ratio of each component (B), (C), (D), and (E) is not particularly limited, but is preferably 10 to 500 parts by mass of the component (B) organopolysiloxane, 10 to 1000 parts by mass of the component (C) organopolysiloxane, 10 to 500 parts by mass of the component (D) monohydric or polyhydric alcohol, and 10 to 800 parts by mass of the component (E) water relative to 10 parts by mass of the component (A) anionic surfactant.

### [Method for Manufacturing Microemulsion Addition-Cured Composition]

The inventive microemulsion composition is preferably addition-cured by a hydrosilylation reaction by adding (F) a hydrosilylation catalyst to the above-described microemulsion composition. The (F) hydrosilylation catalyst can be added after the above-described emulsification step of the microemulsion composition.

There is preferably 0.5 to 3.0 mol of the hydrosilyl groups contained in the (B) relative to 1 mol of the olefinic unsaturated groups contained in the (C). When the amount of the hydrosilyl groups is 0.5 mol or more, the subsequent addition-curing reaction progresses sufficiently, and sufficient feeling can be achieved. In addition, when the amount of the hydrosilyl groups is 3.0 mol or less, the transparency of the microemulsion becomes favorable.

The hydrosilylation reaction is preferably performed in the presence of a platinum group metal-based catalyst such as a platinum catalyst or a rhodium catalyst, etc. As the (F) hydrosilylation catalyst, a chloroplatinic acid, an alcohol-modified chloroplatinic acid, an chloroplatinic acid-vinylsiloxane complex, etc. are preferable, for example. Furthermore, regarding the amount of the catalyst used, the amount of platinum or rhodium is preferably 50 ppm or less, particularly preferably 20 ppm or less relative to the total amount of the microemulsion composition. When the amount of the catalyst used is as described, coloring of the sample due to an excess amount being contained can be suppressed, and transparency becomes favorable.

Since the above-mentioned metal-based catalysts are hydrophobic, the curing reaction rate is sometimes slow when added directly in an oil-in-water type (aqueous micelle solution phase) microemulsion composition. Therefore, it is preferable to raise the reaction rate by coating the catalyst with a dispersant such as a nonionic surfactant. On the other hand, a microemulsion that has a bicontinuous structure does not require a coating with the above-mentioned nonionic surfactant, since the water and the oil are both continuous phases. Therefore, the reaction can be progressed easily just by adding a metal-based catalyst.

The platinum group metal-based catalyst can be added after the emulsification step as described above, but can also be dissolved with the component (B) and the component (C) beforehand. When the platinum group metal-based catalyst is added after the emulsification step, it is possible to dissolve in a solvent and then add. In addition, when dispersibility in water is poor, it is preferable to add the platinum group metal-based catalyst in a state of being dissolved in a nonionic surfactant. When the platinum group metal-based catalyst is dissolved with the component (B) and the component (C) beforehand, it is preferable to keep cooled to a low temperature of 5°C or lower, for example, so that an addition reaction does not occur before the emulsification step is ended.

The addition reaction can be performed at room temperature, for example 20 to 25°C. The stirring time for the reaction is not particularly limited, but is usually 1 to 24 hours. When the reaction does not complete, the reaction can be performed under heating at lower than 100°C. By performing the reaction in such a temperature range, the structure of the emulsion can be prevented more certainly from collapsing.

In addition, the present invention provides a microemulsion addition-cured composition that has a transparent or translucent appearance even after addition-curing. A microemulsion addition-cured composition obtained by addition-curing a microemulsion composition containing the above-described (B) and (C) can suppress influence to the phases of the emulsion due to generation of heat or contraction of the structure that accompany the addition-curing reaction, and the transparency after the reaction becomes favorable.

### [Physical Properties of Microemulsion Addition-Cured Composition]

By blending the inventive microemulsion addition-cured composition in a cosmetic as a feeling improver, stickiness arising from a surfactant is reduced, and a cosmetic excellent in feeling on use, refreshing feeling, and temporal stability can be achieved.

In addition, in the present invention, it is possible to produce a microemulsion addition-cured composition having a bicontinuous structure as an intermediate composition in view of convenience in blending in an aqueous type or an emulsion type composition. That is, water can be added to the inventive microemulsion addition-cured composition having a bicontinuous structure to once prepare a water-dispersion emulsion dispersed in an aqueous phase as an intermediate composition, and a cosmetic with this intermediate composition blended can be prepared.

### [Cosmetics]

The inventive microemulsion addition-cured composition can be used for various uses, and in particular, is applicable as a raw material of all cosmetics externally used for the skin or hair. In this case, the blended amount of the microemulsion addition-cured composition is preferably 0.1 to 40 mass% of the total cosmetic, further preferably 0.1 to 10 mass%.
With 0.1 mass% or more, sufficient feeling can be achieved, and with 40 mass% or less, feeling on use becomes favorable.

### [Other Components]

The inventive microemulsion addition-cured composition and cosmetics containing the inventive microemulsion addition-cured composition may be blended with various other components used in usual cosmetics. Examples of the other components include, for example, an oil agent other than components (B) and (C) as (G), (H) a powder, (I) a surfactant other than the component (A), (J) a crosslinked organopolysiloxane, (K) a film former, and (L) other additives. One of these can be used or an appropriate combination of two or more thereof can be used. These components are appropriately selected for use depending on the kind of the cosmetic, and so on. The amount of these components to be blended can be a known amount which depends on the kind of the cosmetic, and so on.

### (G): Oil agents other than components (B) and (C)

One or more oil agents selected from oil agents (G) other than components (B) and (C) can be blended in the inventive cosmetic according to the object. An oil agent in any form of solid, semi-solid, or liquid can be used as long as it is used in usual cosmetics. For example, natural vegetable and animal fats and oils, semi-synthetic fats and oils, hydrocarbon oils, higher alcohols, ester oils, commonly used silicone oils, fluorinated oil agents, ultraviolet absorbers, and the like can be used. In a case where an oil agent is blended, the amount of the oil agent blended is not particularly limited, but is preferably 1 to 95 mass%, more preferably 1 to 30 mass% of the total cosmetic.

### · Silicone oils

Examples of the silicone oils include low viscous to high viscous linear or branched organopolysiloxanes such as dimethyl polysiloxane, tristrimethylsiloxy methylsilane, caprylyl methicone, phenyl trimethicone, tetrakistri methylsiloxysilane, methylphenylpolysiloxane, methylhexylpolysiloxane, methyl hydrogen polysiloxane, and dimethylsiloxane/methylphenylsiloxane copolymers; cyclic organopolysiloxanes such as octamethyl cyclotetrasiloxane, decamethyl cyclopentasiloxane, dodecamethyl cyclohexasiloxane, tetramethyl tetrahydrogen cyclotetrasiloxane, and tetramethyltetraphenyl cyclotetrasiloxane; silicone rubbers such as amino-modified organopolysiloxanes, pyrrolidone-modified organopolysiloxanes, pyrrolidone carboxylate-modified organopolysiloxanes, gum dimethyl polysiloxanes with high polymerization degree, gum amino-modified organopolysiloxanes, and gum dimethylsiloxane/methylphenylsiloxane copolymers; silicone gum and rubber cyclic organopolysiloxane solutions, higher alkoxy-modified silicone such as stearoxysilicone, higher fatty acid-modified silicones, alkyl-modified silicones, long chain alkyl-modified silicones, amino acid-modified silicones, fluorine-modified silicones, and the like.

### · Natural vegetable and animal fats and oils and semi-synthetic fats and oils

Examples of the natural animal and vegetable oils and fats and semi-synthetic oils and fats include avocado oil, linseed oil, almond oil, insects wax, perilla oil, olive oil, cacao butter, kapok wax, kaya oil, carnauba wax, liver oil, candelilla wax, purified candelilla wax, beef tallow, neats foot fat, beef bone fat, hardened beef tallow, apricot kernel oil, whale wax, hardened oil, wheat germ oil, sesame oil, rice germ oil, rice bran oil, sugarcane wax, Camellia sasanqua oil, safflower oil, shea butter, Chinese tung oil, cinnamon oil, jojoba wax, squalane, squalene, shellac wax, turtle oil, soybean oil, tea seed oil, camellia oil, evening primrose oil, corn oil, lard, rapeseed oil, Japanese tung oil, bran wax, germ oil, horse fat, persic oil, palm oil, palm kernel oil, castor oil, hardened castor oil, castor oil fatty acid methyl ester, sunflower oil, grape oil, bayberry wax, jojoba oil, macadamia nut oil, bees wax, mink oil, meadowfoam oil, cottonseed oil, cotton wax, Japan wax, Japan wax kernel oil, montan wax, coconut oil, hardened coconut oil, tri-coconut oil fatty acid glyceride, mutton tallow, peanut oil, lanolin, liquid lanolin, reduced lanolin, lanolin alcohol, hard lanolin, lanolin acetate, acetylated lanolin alcohol, lanolin fatty acid isopropyl, POE lanolin alcohol ether, POE lanolin alcohol acetate, lanolin fatty acid polyethylene glycol, POE hydrogenated lanolin alcohol ether, egg yolk oil, etc. Provided that POE means polyoxyethylene.

### · Hydrocarbon oils

Examples of the hydrocarbon oils include a linear, branched, and further volatile hydrocarbon oils, etc., and specific examples include ozokerite, α-olefin oligomer, light isoparaffin, isododecane, isohexadecane, light liquid isoparaffin, squalane, synthetic squalane, vegetable squalane, squalene, ceresin, paraffin, paraffin wax, polyethylene wax, polyethylene·polypropylene wax, an (ethylene/propylene/styrene) copolymer, a (butylene/propylene/styrene) copolymer, liquid paraffin, liquid isoparaffin, pristane, polyisobutylene, hydrogenated polyisobutene, microcrystalline wax, vaseline, higher fatty acid, etc. Examples of the higher fatty acid include lauric acid, myristic acid, palmitic acid, stearic acid, behenic acid, undecylenic acid, oleic acid, linoleic acid, linolenic acid, arachidonic acid, eicosapentaenoic acid (EPA), docosahexaenoic acid (DHA), isostearic acid, 12-hydroxystearic acid, etc.

### · Higher alcohols

Examples of the higher alcohols includes lauryl alcohol, myristyl alcohol, palmityl alcohol, stearyl alcohol, behenyl alcohol, hexadecyl alcohol, oleyl alcohol, isostearyl alcohol, hexyldodecanol, octyldodecanol, cetostearyl alcohol, 2-decyltetradecynol, cholesterol, phytosterol, POE cholesterol ether, monostearyl glycerin ether (batyl alcohol), monooleyl glyceryl ether (selacyl alcohol), etc.

### · Ester oils

Examples of the ester oils include diisobutyl adipate, 2-hexyldecyl adipate, di-2-heptylundecyl adipate, N-alkylglycol monoisostearate, isocetyl isostearate, trimethylolpropane triisostearate, ethylene glycol di-2-ethylhexanoate, cetyl 2-ethylhexanoate, trimethylolpropane tri-2-ethylhexanoate, pentaerythritol tetra-2-ethylhexanoate, cetyl octanoate, octyldodecyl gum ester, oleyl oleate, octyldodecyl oleate, decyl oleate, neopentyl glycol dioctanoate, neopentyl glycol dicaprate, triethyl citrate, 2-ethylhexyl succinate, amyl acetate, ethyl acetate, butyl acetate, isocetyl stearate, butyl stearate, diisopropyl sebacate, di-2-ethylhexyl sebacate, cetyl lactate, myristyl lactate, isononyl isononanate, isotridecyl isononanate, isopropyl palmitate, 2-ethylhexyl palmitate, 2-hexyldecyl palmitate, 2-heptylundecyl palmitate, cholesteryl 12-hydroxystearate, dipentaerythritol fatty acid ester, isopropyl myristate, octyldodecyl myristate, 2-hexyldecyl myristate, myristyl myristate, hexyldecyl dimethyloctanoate, ethyl laurate, hexyl laurate, N-lauroyl-L-glutamic acid-2-octyldodecyl ester, isopropyl lauroyl sarcosinate, diisostearyl malate, and glyceride oil, etc. Examples of the glyceride oil include acetoglyceryl, glyceryl triisooctanoate, glyceryl triisostearate, glyceryl triisopalmitate, glyceryl tribehenate, glyceryl monostearate, glyceryl di-2-heptylundecanoate, glyceryl trimyristate, diglyceryl isostearate/myristate, etc.

### · Fluorinated oil agents

Examples of the fluorinated oil agents include perfluoropolyether, perfluorodecalin, perfluorooctane, etc.

### · Ultraviolet absorbers

Examples of the ultraviolet absorbers include a benzoic acid-based ultraviolet absorber such as para-aminobenzoic acid, etc., an anthranilic acid-based ultraviolet absorber such as methyl anthranilate, etc., a salicylic acid-based ultraviolet absorber such as methyl salicylate, octyl salicylate, trimethylcyclohexylhexyl salicylate, etc., a cinnamic acid-based ultraviolet absorber such as octyl para-methoxycinnamate, etc., a benzophenone-based ultraviolet absorber such as 2,4-dihydroxybenzophenone, etc., an urocanic acid-based ultraviolet absorber such as ethyl urocanate, etc., a dibenzoylmethane-based ultraviolet absorber such as 4-t-butyl-4'-methoxy-dibenzoylmethane, etc., phenylbenzimidazole sulfonic acid, a triazine derivative, etc. The ultraviolet absorbers may contain an ultraviolet absorptive scattering agent. Examples of the ultraviolet absorptive scattering agent include powder which absorbs or scatters ultraviolet rays such as fine particulate titanium oxide, fine particulate iron-containing titanium oxide, fine particulate zinc oxide, fine particulate cerium oxide and a complex thereof, etc., and a dispersion in which these powders which absorb and scatter ultraviolet rays are dispersed in the oil agent in advance can also be used.

### (H) Powder

As the powder, any of the materials can be used as long as it is used for the usual cosmetics, regardless of its shape (spherical, needle-like, plate-like, etc.) or particle diameter (fumed, fine particles, pigment grade, etc.), particulate structure (porous, nonporous, etc.). Examples include silicone spherical powder, inorganic powder, organic powder, surfactant metal salt powder, colored pigment, pearl pigment, metal powder pigment, tar pigment, natural pigment, etc.

### · Inorganic powder

Specific examples of the inorganic powder include powders selected from titanium oxide, zirconium oxide, zinc oxide, cerium oxide, magnesium oxide, barium sulfate, calcium sulfate, magnesium sulfate, calcium carbonate, magnesium carbonate, talc, mica, kaolin, sericite, muscovite, synthetic mica, phlogopite, lepidolite, biotite, lithia mica, silicic acid, silicic anhydride, aluminum silicate, magnesium silicate, magnesium aluminum silicate, calcium silicate, barium silicate, strontium silicate, metal tungstate, hydroxyapatite, vermiculite, Higilite, bentonite, montmorillonite, hectorite, zeolite, ceramic powder, dibasic calcium phosphate, alumina, aluminum hydroxide, boron nitride, boron nitride, silica, etc.

### · Organic powder

Examples of the organic powder include powders selected from polyamide powder, polyester powder, polyethylene powder, polypropylene powder, polystyrene powder, polyurethane, benzoguanamine powder, polymethylbenzoguanamine powder, tetrafluoroethylene powder, polymethyl methacrylate powder, cellulose, silk powder, Nylon powder, 12 Nylon, 6 Nylon, silicone powder, styrene·acrylic acid copolymer, divinylbenzene-styrene copolymer, vinyl resin, urea resin, phenol resin, fluorine resin, silicon resin, acrylic resin, melamine resin, epoxy resin, polycarbonate resin, microcrystalline fiber powder, starch powder, lauroyl lysine, etc.

### · Surfactant metal salt powder

Examples of the surfactant metal salt powder (metallic soap) include powders selected from zinc stearate, aluminum stearate, calcium stearate, magnesium stearate, zinc myristate, magnesium myristate, zinc cetyl phosphate, calcium cetyl phosphate, zinc sodium cetyl phosphate, etc.

### · Colored pigment

Examples of the colored pigment include powders selected from inorganic red pigments such as iron oxide, iron hydroxide and iron titanate, inorganic brown pigments such as γ-iron oxide, etc., inorganic yellow pigments such as yellow iron oxide, loess, etc., inorganic black pigments such as black iron oxide, carbon black, etc., inorganic violet pigments such as manganese violet, cobalt violet, etc., inorganic green pigments such as chromium hydroxide, chromium oxide, cobalt oxide, cobalt titanate, etc., inorganic blue pigments such as prussian blue, ultramarine blue, etc., those obtained by laking tar pigments, those obtained by laking natural dyes, and synthetic resin powders obtained by combining these powders, etc.

### · Pearl pigment

Examples of the pearl pigment include powders selected from titanium oxide-coated mica, titanium oxide-coated mica, bismuth oxychloride, titanium oxide-coated bismuth oxychloride, titanium oxide-coated talc, fish scale foil, titanium oxide-coated colored mica, etc.

### · Metal powder pigment

Examples of the metal powder pigment include powders selected from aluminum powder, copper powder, stainless powder, etc.

### · Tar pigment

Examples of the tar pigment include powders selected from Red No. 3, Red No. 104, Red No. 106, Red No. 201, Red No. 202, Red No. 204, Red No. 205, Red No. 220, Red No. 226, Red No. 227, Red No. 228, Red No. 230, Red No. 401, Red No. 505, Yellow No. 4, Yellow No. 5, Yellow No. 202, Yellow No. 203, Yellow No. 204, Yellow No. 401, Blue No. 1, Blue No. 2, Blue No. 201, Blue No. 404, Green No. 3, Green No. 201, Green No. 204, Green No. 205, Orange No. 201, Orange No. 203, Orange No. 204, Orange No. 206, Orange No. 207, etc.

### · Natural pigment

Examples of the natural pigment include powders selected from carminic acid, laccaic acid, carthamin, brazilin, crocin, etc.

As these powders, those in which powders are compounded, or those treated with general oil, silicone oil, a fluorine compound, a surfactant, etc., may also be used. One or more of those treated with a hydrolyzable silyl group or an alkyl group having a hydrogen atom directly bonded to a silicon atom, a linear and/or branched organopolysiloxane having a hydrolyzable silyl group or a hydrogen atom directly bonded to a silicon atom, a linear and/or branched organopolysiloxane having a hydrolyzable silyl group or a hydrogen atom directly bonded to a silicon atom and being co-modified by a long chain alkyl, a linear and/or branched organopolysiloxane having a hydrolyzable silyl group or a hydrogen atom directly bonded to a silicon atom and being co-modified by polyoxyalkylene, an acrylic-silicone-based copolymer having a hydrolyzable silyl group or a hydrogen atom directly bonded to a silicon atom, etc., may also be used depending on necessity. A silicone treatment agent is more preferable, and examples thereof include silanes or silylation agents such as caprylylsilane (AES-3083 manufactured by Shin-Etsu Chemical Co., Ltd.) or trimethoxysilyl dimethicone, etc., silicone oils such as dimethyl silicone (KF-96A series manufactured by Shin-Etsu Chemical Co., Ltd.), methyl hydrogen polysiloxane (KF-99P, KF-9901, etc. manufactured by Shin-Etsu Chemical Co., Ltd.), silicone-branched silicone treatment agent (KF-9908, KF-9909, etc. manufactured by Shin-Etsu Chemical Co., Ltd.) etc., and acrylic silicone (KP-574 and KP-541 manufactured by Shin-Etsu Chemical Co., Ltd.), etc. Specific examples of pigments with a surface treatment include the KTP-09 series manufactured by Shin-Etsu Chemical Co., Ltd., in particular, KTP-09W, 09R, 09Y, 09B, etc. Specific examples of dispersions containing hydrophobized fine-particle titanium oxide or hydrophobized fine-particle zinc oxide include SPD-T5, T6, T7, T5L, Z5, Z6, Z5L, etc. manufactured by Shin-Etsu Chemical Co., Ltd.

### · Silicone spherical powder

Examples of the silicone spherical powder include crosslinked silicone powders (i.e., what is called silicone rubber powders of organopolysiloxanes having such a structure that repeating chains of diorganosiloxane units are crosslinked), silicone resin particles (polyorganosilsesquioxane resin particles having a three-dimensional network structure), silicone resin-coated silicone rubber powders, etc.

Specific examples of the crosslinked silicone powders and silicone resin particles include those known under names such as (dimethicone/vinyl dimethicone) crosspolymer and polymethylsilsesquioxane, etc. These are commercially available as powder or swollen material containing silicone oil under product names such as, for example, KMP-598, 590, 591, and KSG-016F (all of which are manufactured by Shin-Etsu Chemical Co., Ltd.). These powders provide cosmetics with smoothness by a rolling effect peculiar to spherical powders, and improve feeling on use. One of these can be used or a combination of two or more thereof can be used.

Silicone resin-coated silicone rubber powders are particularly favorable since silicone resin-coated silicone rubber powders have the effect of improving feeling, for example, preventing stickiness, etc. and the effect of correcting unevenness of wrinkles and pores, etc. and the like. As specific examples of the silicone resin-coated silicone rubber powders, those such as (vinyl dimethicone/methicone silsesquioxane) crosspolymer, (diphenyl dimethicone/vinyldiphenyl dimethicone/silsesquioxane) crosspolymer, polysilicone-22, and polysilicone-1 crosspolymers, etc. can be used, which are defined in Cosmetic-Info.jp. These are commercially available under product names such as KSP-100, 101, 102, 105, 300, 411, and 441 (all of which are manufactured by Shin-Etsu Chemical Co., Ltd.). One of these powders can be used or a combination of two or more thereof can be used.

When a powder is blended, the amount of the powder blended is not particularly limited, but 0.1 to 90 mass% of the total cosmetic is preferably blended, further preferably 1 to 35 mass%.

### (I) Surfactant other than component (A)

The surfactant other than the component (A) includes nonionic, cationic and amphoteric surfactants, but is not particularly limited, and any of these can be used as long as it is used in usual cosmetics. One of these can be used or an appropriate combination of two or more thereof can be used.

Among these surfactants, preferable are crosslinked polyether-modified silicones, crosslinked polyglycerin-modified silicones, linear or branched polyoxyethylene-modified organopolysiloxanes, linear or branched polyoxyethylene-polyoxypropylene-modified organopolysiloxanes, linear or branched polyoxyethylene/alkyl-co-modified organopolysiloxanes, linear or branched polyoxyethylene-polyoxypropylene/alkyl-co-modified organopolysiloxanes, linear or branched polyglycerin-modified organopolysiloxanes, and linear or branched polyglycerin/alkyl-co-modified organopolysiloxanes, in view of compatibility with oil agents containing the component (A).

In these surfactants, the content of hydrophilic polyoxyethylene groups, polyoxyethylene-polyoxypropylene groups, or polyglycerin residues is preferably 10 to 70% in the molecule. Specific examples of such surfactants include KSG-210, 240, 310, 320, 330, 340, 320Z, 350Z, 710, 810, 820, 830, 840, 820Z, 850Z, KF-6011, 6013, 6017, 6043, 6028, 6038, 6048, 6100, 6104, 6105, and 6106, manufactured by Shin-Etsu Chemical Co., Ltd., and the like.

When the component (I) is blended, the blended amount is preferably 0.01 to 15 mass% in the cosmetic.

### (J) Crosslinked organopolysiloxane

The crosslinked organopolysiloxane is not particularly limited as long as it is used in usual cosmetic products. One of the crosslinked organopolysiloxane can be used or an appropriate combination of two or more thereof can be used.

Unlike the silicone spherical powders described in (H) above, the crosslinked organopolysiloxane does not have a spherical shape.

In addition, unlike the (I) surfactant other than the component (A), the component (J) is preferably a compound having no polyether- or polyglycerin structure in the molecular structure, and is an elastomer having structural viscosity by swelling with the oil agent. Specific examples of the crosslinked organopolysiloxane include (dimethicone/vinyl dimethicone) crosspolymers, (dimethicone/phenylvinyl dimethicone) crosspolymers, (vinyl dimethicone/lauryl dimethicone) crosspolymers, (lauryl polydimethylsiloxyethyl dimethicone/bis-vinyl dimethicone) crosspolymers, and the like, which are defined in Cosmetic-Info.jp. These are commercially available as swollen materials containing oil which is liquid at room temperature. Specific examples thereof include KSG-15, 1510, 16, 1610, 18A, 19, 41A, 42A, 43, 44, 042Z, 045Z, and 048Z, which are manufactured by Shin-Etsu Chemical Co., Ltd., and the like.

When the component (J) is blended, the blended amount is preferably 0.01 to 30 mass% in the cosmetic as solid contents.

### (K) Film former

As the film former, existing film formers can be used in combination. The existing film formers are not particularly limited as long as the raw material can be blended in usual cosmetics. Specifically, used as the film former are: latexes such as polyvinyl alcohol, polyvinylpyrrolidone, polyvinyl acetate, and polyalkyl acrylate; cellulose derivatives such as dextrin, alkyl cellulose and nitrocellulose; siliconized polysaccharides such as pullulan tri(trimethylsiloxy)silylpropylcarbamate; acrylic-silicone graft copolymers such as (alkyl acrylate/dimethicone) copolymers; silicone resins such as trimethylsiloxysilicate; silicone-based resins such as silicone-modified polynorbornene and fluorine-modified silicone resins; fluorinated resins, aromatic hydrocarbon resins, polymer emulsion resins, terpene-based resins, polybutene, polyisoprene, alkyd resins, polyvinylpyrrolidone-modified polymers, rosin-modified resins, polyurethanes, and the like.

Among these, silicone-based film formers are particularly preferable. Above all, it is possible to use, without limitation to, pullulan tri(trimethylsiloxy)silylpropyl carbamate (commercially available products, dissolved in a solvent, include TSPL-30-D5 and ID manufactured by Shin-Etsu Chemical Co., Ltd.), (alkyl acrylate/dimethicone) copolymers (commercially available products, dissolved in a solvent, include KP-543, 545, 549, 550, and 545L manufactured by Shin-Etsu Chemical Co., Ltd., and the like), trimethylsiloxysilicate (commercially available products, dissolved in a solvent, include KF-7312J and X-21-5250 manufactured by Shin-Etsu Chemical Co., Ltd., and the like), silicone-modified polynorbornene (commercially available products, dissolved in a solvent, include NBN-30-ID manufactured by Shin-Etsu Chemical Co., Ltd., and the like), an organosiloxane graft polyvinyl alcohol polymer, and the like.

When the component (K) is blended, the blended amount is preferably 0.1 to 20 mass% in the cosmetic.

### (L) Other additives

Examples of the other additives include an oil-soluble gelling agent, water-soluble thickening agent, antiperspirant, preservative and antimicrobial, perfume, salt, antioxidant, pH adjuster, chelator, refrigerant, anti-inflammatory agent, skincare component (such as whitening agent, cell activator, rough skin improver, blood circulation promoter, skin astringent, antiseborrheic agent), vitamin, amino acid, nucleic acid, hormone, inclusion compound, and the like. One of these components (L) can be used or an appropriate combination of two or more thereof can be used. When the component (L) is blended, the blended amount is preferably 0.1 to 20 mass% in the cosmetic.

### · Oil-soluble gelling agent

The oil-soluble gelling agent includes metal soaps such as aluminum stearate, magnesium stearate, and zinc myristate; amino acid derivatives such as N-lauroyl-L-glutamic acid and α,γ-di-n-butylamine; dextrin fatty acid esters such as dextrin palmitic acid ester, dextrin stearic acid ester, and dextrin 2-ethylhexanoic acid/palmitic acid ester; sucrose fatty acid esters such as sucrose palmitic acid ester and sucrose stearic acid ester; fructo-oligosaccharide fatty acid esters such as fructo-oligosaccharide stearic acid ester and fructo-oligosaccharide 2-ethylhexanoic acid ester; benzylidene derivatives of sorbitol such as monobenzylidene sorbitol and dibenzylidene sorbitol; organic-modified clay minerals of disteardimonium hectorite, stearalkonium hectorite, and hectorite; and the like.

### · Water-soluble thickening agent

Examples of the water-soluble thickening agent include plant polymers such as an Arabia gum, tragacanth, galactan, a carob gum, a guar gum, a karaya gum, carrageenan, pectin, agar, quince seed (marmelo), starch (rice, corn, potato, wheat, and so on), an algae colloid, a trant gum, a locust bean gum; microbial polymers such as a xanthan gum, dextran, succinoglucan, and pullulan; animal polymers such as collagen, casein, albumin, and gelatin; starch polymers such as carboxymethyl starch and methyl hydroxypropyl starch; cellulose polymers such as methyl cellulose, ethyl cellulose, methyl hydroxypropyl cellulose, carboxymethyl cellulose, hydroxymethyl cellulose, hydroxypropyl cellulose, nitrocellulose, sodium cellulose sulfate, sodium carboxymethyl cellulose, crystalline cellulose, cationized cellulose, and cellulose powder; alginic acid polymers such as sodium alginate and propylene glycol alginate ester; vinyl polymers such as polyvinyl methyl ether and carboxy vinyl polymer; a polyoxyethylene polymer; polyoxyethylene polyoxypropylene copolymer polymers; acryl polymers such as sodium polyacrylate, polyethyl acrylate, polyacrylamide, and an acryloyldimethyl taurate salt copolymer; other synthetic water-soluble polymers such as polyethyleneimine and a cationic polymer; inorganic water-soluble polymers such as a bentonite, aluminum magnesium silicate, montmorillonite, beidellite, nontronite, saponite, hectorite, anhydrous silicic acid; and the like.

Among them, one or a combination of two or more water-soluble thickening agents selected from plant polymers, microbial polymers, animal polymers, starch polymers, cellulose polymers, alginic acid polymers, polyoxyethylene polyoxypropylene copolymer polymers, acryl polymers, and inorganic water-soluble polymers are preferably used.

### · Antiperspirant

Examples of the antiperspirant include aluminum hydroxyhalides such as chlorohydroxy aluminum and aluminum chlorohydroxy allantoinate; aluminum halides such as aluminum chloride; aluminum allantoinate, tannic acid, persimmon tannin, potassium aluminum sulfate, zinc oxide, zinc para-phenolsulfonate, burnt alum, aluminum zirconium tetrachlorohydrate, aluminum zirconium trichlorohydrex glycine, and the like. In particular, as components that exhibit a high effect, aluminum hydroxyhalide, aluminum halide, and a complex or mixture thereof with zirconyl oxyhalide and zirconyl hydroxyhalide (for example, aluminum zirconium tetrachlorohydrate and aluminum zirconium trichlorohydrex glycine), and the like are preferable.

### · Preservative and antimicrobial

The preservative and antimicrobial include para-oxybenzoate alkyl ester, benzoic acid, sodium benzoate, sorbic acid, potassium sorbate, phenoxy ethanol, imidazolidinyl urea, salicylic acid, isopropylmethylphenol, carbolic acid, p-chloro-m-cresol, hexachlorophene, benzalkonium chloride, chlorhexidine chloride, trichlorocarbanilide, iodopropynyl butylcarbamate, polylysine, photosensitizers, silver, plant extracts, and the like.

### · Perfume

Examples of the perfume include natural perfumes and synthetic perfumes. Examples of the natural perfume include vegetable perfume separated from flowers, leaves, wood, pericarp, etc.; and animal perfume such as musk, civet, etc. Examples of the synthetic perfume include hydrocarbons such as monoterpene, etc.; alcohols such as an aliphatic alcohol, an aromatic alcohol, etc.; aldehydes such as terpene aldehyde, aromatic aldehyde, etc.; ketones such as an alicyclic ketone, etc.; esters such as a terpene-based ester, etc.; lactones; phenols; oxides; nitrogen-containing compounds; acetals, etc.

### · Salt

Examples of the salt include an inorganic salt, an organic acid salt, an amine salt and an amino acid salt. Examples of the inorganic salt include a sodium salt, a potassium salt, a magnesium salt, a calcium salt, an aluminum salt, a zirconium salt, a zinc salt, etc., of an inorganic acid such as hydrochloric acid, sulfuric acid, carbonic acid, nitric acid, etc. Examples of the organic acid salt include salts of an organic acid such as acetic acid, dehydroacetic acid, citric acid, malic acid, succinic acid, ascorbic acid, stearic acid, etc. Examples of the amine salt and the amino acid salt include a salt of an amine such as triethanolamine, etc., and a salt of an amino acid such as glutamic acid, etc. In addition, as others, a salt of hyaluronic acid, chondroitin sulfuric acid, etc., and further an acid-alkali neutralizing salt used in preparation prescription can be also used.

### · Antioxidant

Examples of the antioxidant include, but are not particularly limited to, carotenoid, ascorbic acid and a salt thereof, ascorbyl stearate, tocopherol acetate, tocopherol, p-t-butylphenol, butylhydroxyanisol, dibutylhydroxytoluene, phytic acid, ferulic acid, thiotaurine, hypotaurine, sulfite, erythorbic acid and a salt thereof, chlorogenic acid, epicatechin, epigallocatechin, epigallocatechin gallate, apigenin, campherol, myricetin, quercetin, and the like.

### · pH adjuster

Examples of the pH adjuster include lactic acid, citric acid, glycolic acid, succinic acid, tartaric acid, dl-malic acid, potassium carbonate, sodium bicarbonate, ammonium bicarbonate, and the like.

### · Chelator

Examples of the chelator include alanine, sodium edetate, sodium polyphosphate, sodium metaphosphate, phosphoric acid, and the like.

### · Refrigerant

Examples of the refrigerant include L-menthol, camphor, menthyl lactate, and the like.

### · Anti-inflammatory agent

Examples of the anti-inflammatory agent include allantoin, glycyrrhizinic acid and a salt thereof, glycyrrhetinic acid and stearyl glycyrrhetinate, tranexamic acid, azulene, and the like.

### · Skincare component

Examples of the skincare component include a skin-brightening agent such as a placenta extract, arbutin, glutathione, and strawberry geranium extract; a cell activator such as royal jelly, a photosensitizer, a cholesterol derivative, and a calf blood extract; a rough skin-improving agent, a blood circulation promoter such as vanillylamide nonylate, benzyl nicotinate, β-butoxyethyl nicotinate, capsaicin, zingerone, cantharides tincture, ichthammol, caffeine, tannic acid, α-borneol, tocopherol nicotinate, inositol hexanicotinate, cyclandelate, cinnarizine, tolazoline, acetylcholine, verapamil, cepharanthine, and γ-orizanol; a skin astringent, an antiseborrheic agent such as sulfur and thianthrol, and the like.

### · Vitamin

Examples of the vitamin include vitamin A such as vitamin A oil, retinol, retinol acetate, and retinol palmitate; vitamin B including vitamin B₂ such as riboflavin, riboflavin butyrate, and flavin adenine nucleotide, vitamin B₆ such as pyridoxine hydrochloride, pyridoxine dioctanoate, and pyridoxine tripalmitate, vitamin B₁₂ and a derivative thereof, and vitamin B₁₅ and a derivative thereof; vitamin C such as L-ascorbic acid, L-ascorbic acid dipalmitate, sodium L-ascorbic acid-2-sulfate, and dipotassium L-ascorbic acid diphosphate; vitamin D such as ergocalciferol and cholecalciferol; vitamin E such as α-tocopherol, β-tocopherol, γ-tocopherol, dl-α-tocopherol acetate, dl-α-tocopherol nicotinate, and dl-α-tocopherol succinate; nicotinic acids such as nicotinic acid, benzyl nicotinate, and amide nicotinate; pantothenic acids such as vitamin H, vitamin P, calcium pantothenate, D-pantothenyl alcohol, pantothenyl ethyl ether, and acetyl pantothenyl ethyl ether; biotin, and the like.

### · Amino acid

Examples of the amino acid include glycine, valine, leucine, isoleucine, serine, threonine, phenylalanine, arginine, lysine, aspartic acid, glutamic acid, cystine, cysteine, methionine, tryptophan, and the like.

### · Nucleic acid

Examples of the nucleic acid include deoxyribonucleic acid, and the like.

### · Hormone

Examples of the hormone include estradiol, ethenyl estradiol, and the like.

### · Inclusion compound

Examples of the inclusion compound include cyclodextrin, and the like.

The cosmetic per se of the present invention is not particularly limited. For example, the present invention is applicable to various products such as beauty essence, milky lotion, cream, hair care product, foundation, makeup base, sunscreen, concealer, cheek color, lipstick, gloss, balm, mascara, eye shadow, eyeliner, body make-up, deodorant, and manicure product. Among these, make-up cosmetics such as foundation, lipstick, mascara, and eyeliner, etc. and cosmetics provided with a sunscreening effect are particularly preferable.

The physical form of the inventive cosmetic can be selected from various physical forms such as liquid, cream, solid, paste, gel, mousse, souffle, clay, powder, and stick forms.

### EXAMPLE

Hereinafter, the present invention will be described more specifically with reference to Examples of the present invention and Comparative Example. However, the present invention is not limited to the following Examples.

### (Examples 1 to 11)

In a 200-mL glass beaker were charged SFNa (KANEKA Surfactin, Kaneka Corporation) as (A) an anionic surfactant, organopolysiloxanes (1) to (3) shown in the following formulae as (B) an organopolysiloxane having at least two hydrosilyl groups in one molecule thereof, organopolysiloxanes (4) to (6) shown in the following formulae as (C) an organopolysiloxane having at least two olefinic unsaturated groups in one molecule thereof, glycerin, being a polyhydric alcohol, as (D) a monohydric or polyhydric alcohol, a decamethylcyclopentasiloxane (KF-995) or a linear organopolysiloxane (KF-96A-6cs) (a silicone oil having a viscosity of 20 mm²/s or less at 25°C not containing hydrosilyl groups or olefinic unsaturated groups) as (G) an oil agent other than the components (B) and (C), isododecane as a volatile hydrocarbon oil, and 2-ethylhexyl palmitate as an ester oil by the composition shown in Tables 2 and 3. The mixture was stirred and dissolved at room temperature using a disper, and then (E) water was dropped thereto under room temperature to prepare a microemulsion. In Tables 2 and 3, the blended amounts are shown by mass%. In addition, the kinematic viscosity, the vinyl group content, and the hydrosilyl group content of the organopolysiloxanes (1) to (6) are shown in Table 1.
<Organopolysiloxane (1)> 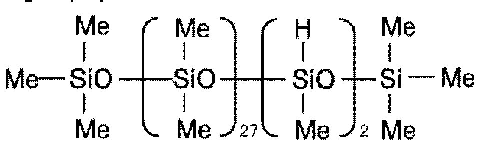
<Organopolysiloxane (2)> 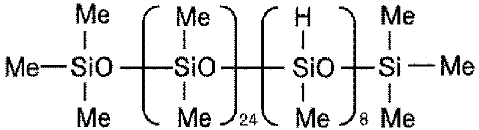
<Organopolysiloxane (3)> 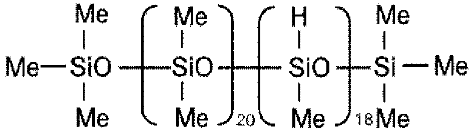
<Organopolysiloxane (4)> 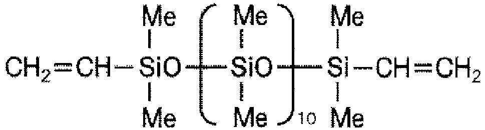
<Organopolysiloxane (5)> 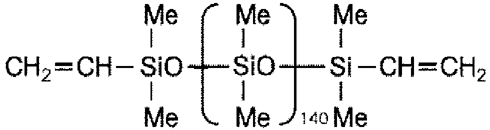
<Organopolysiloxane (6)>

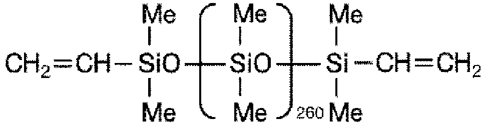

**[Table 1]**

| | Kinematic viscosity mm²/s | Vinyl group content mol/100g | Hydrosilyl group content mol/100g |
|---|---|---|---|
| Organopolysiloxane (1) | 31.9 | - | 0.095 |
| Organopolysiloxane (2) | 26.6 | - | 0.44 |
| Organopolysiloxane (3) | 16.9 | - | 0.75 |
| Organopolysiloxane (4) | 9.2 | 0.20 | - |
| Organopolysiloxane (3) | 385 | 0.018 | - |
| Organopolysiloxane (6) | 567 | 0.015 | - |

The water dispersibility was investigated by adding one drop of the microemulsion prepared in Examples 1 to 11 to 10 ml of water (water dispersibility test). Similarly, the oil dispersibility was investigated by adding one drop of the microemulsion to 10 ml of a D5 (decamethylcyclopentasiloxane) solution (oil dispersibility test). Tables 2 and 3 show the results, along with the evaluation of the appearance of the microemulsions at 25°C.

**[Table 2]**

| | | Example 1 | Example 2 | Example 3 | Example 4 | Example 5 | Example 6 | Example 7 |
|---|---|---|---|---|---|---|---|---|
| Formulated composition (mass%) | SFNa | 1 | 1 | 1 | 1 | 1 | 1 | 1 |
| | Glycerin | 21 | 21 | 21 | 21 | 21 | 21 | 21 |
| | Organo-poly-siloxane (1) | | | | 9. 7 | | | |
| | Organo-poly-siloxane (2) | 2.1 | 2.2 | 2.3 | | | 20.6 | |
| | Organo-poly-siloxane (3) | | | | | 1.4 | | 14.0 |
| | Organo-poly-siloxane (4) | | | | | | 38.4 | 45 |
| | Organo-poly-siloxane (3) | | | | 51.3 | | | |
| | Organo-poly-siloxane (6) | 54.9 | 56.8 | 58.7 | | 57.6 | | |
| | Water | 21 | 19 | 17 | 17 | 19 | 19 | 19 |
| Total | | 100 | 100 | 100 | 100 | 100 | 100 | 100 |
| H/Vi | | 1.1 | 1.1 | 1.1 | 1.1 | 1.1 | 1.1 | 1.1 |
| Appearance of microemulsion | | Translucent | Translucent | Colorless , transparent | Colorless , transparent | Translucent | Translucent | Translucent |
| Water dispersibility test | | Homogeneously dispersed | Homogeneously dispersed | Homogeneously dispersed | Homogeneously dispersed | Homogeneously dispersed | Homogeneously dispersed | Homogeneously dispersed |
| Oil dispersibility test | | Separated | Separated | Separated | Separated | Separated | Separated | Separated |

**[Table 3]**

| | | Example 8 | Example 9 | Example 10 | Example 11 |
|---|---|---|---|---|---|
| Formulated composition (mass %) | SFNa | 1 | 1 | 1 | 1 |
| | Glycerin | 21 | 21 | 21 | 21 |
| | Organopolysiloxane (2) | 1.9 | 1.9 | 1.9 | 1.9 |
| | Organopolysiloxane (6) | 48.9 | 48.9 | 48.9 | 48.9 |
| | KF-995 | 10.2 | | | |
| | KF-96A-6cs | | 10.2 | | |
| | Isododecane | | | 10.2 | |
| | 2-ethylhexyl palmitate | | | | 10.2 |
| | Water | 17 | 17 | 17 | 17 |
| Total | | 100 | 100 | 100 | 100 |
| H/Vi | | 1.1 | 1.1 | 1.1 | 1.1 |
| Appearance of microemulsion | | Colorless, transparent | Colorless, transparent | Translucent | Translucent |
| Water dispersibility test | | Homogeneously dispersed | Homogeneously dispersed | Homogeneously dispersed | Homogeneously dispersed |
| Oil dispersibility test | | Separated | Separated | Separated | Separated |

As shown in the above Tables 2 and 3, microemulsions having a transparent or translucent appearance at 25°C were achieved in Examples 1 to 11. When the microemulsions were dropped into water, each microemulsion dispersed homogeneously, and thus, dispersibility in water was confirmed. On the other hand, when the microemulsion were dropped into oil, each microemulsion was separated, and was not dispersed in the oil.

A pigment solubility test was conducted on microemulsions (Examples 3, 4, 6, and 8 to 10) obtained in a colorless and transparent or translucent state. As test methods, the water solubility was investigated by adding an aqueous water-soluble pigment (Blue #1) solution (concentration: 0.1 mass%) to the obtained microemulsion. Meanwhile, the oil solubility was investigated in the same manner as above by adding an oil-soluble pigment (β-carotene) solution in decamethylcyclopentasiloxane (concentration: 1.0 mass%) to the obtained microemulsion. Both the water-soluble pigment and the oil-soluble pigment were dissolved homogeneously, and thus, it was confirmed that the microemulsions had a bicontinuous structure. Table 4 shows the results.

**[Table 4]**

| | | Example 3 | Example 4 | Example 6 | Example 8 | Example 9 | Example 10 |
|---|---|---|---|---|---|---|---|
| Appearance of microemulsion | | Colorless , transparent | Colorless , transparent | Translucent | Colorless , transparent | Colorless , transparent | Translucent |
| Pigment solubility test | Water solubility | Homogeneously dissolved | Homogeneously dissolved | Homogeneously dissolved | Homogeneously dissolved | Homogeneously dissolved | Homogeneously dissolved |
| | Oil solubility | Homogeneously dissolved | Homogeneously dissolved | Homogeneously dissolved | Homogeneously dissolved | Homogeneously dissolved | Homogeneously dissolved |

### (Examples 12 to 17)

While maintaining a 200-mL glass beaker containing 100 g of the microemulsions of Examples 3, 4, 6, and 8 to 10 having a transparent or translucent appearance obtained above at 20 to 25°C on a stirring apparatus, 0.1 g (5 ppm of platinum relative to the total amount of the microemulsion composition) of a solution of a chloroplatinic acid-vinylsiloxane complex in toluene (platinum concentration: 0.5 wt%) was added while stirring, and the resultant was stirred for 12 hours within the same temperature range as above. Thus, an addition-curing reaction of the (B) organopolysiloxane having hydrosilyl groups and the (C) organopolysiloxane having vinyl groups was performed to produce a microemulsion addition-cured composition. Progression of the reaction was confirmed by NMR measurement, since the peak attributable to vinyl groups had disappeared in each case. Table 5 shows the evaluation of the appearance of the microemulsions before the reaction and addition-cured materials thereof at 25°C, and the results of a pigment solubility test, a water dispersibility test, and an oil dispersibility test conducted in the same manner as in Examples 3, 4, 6, and 8 to 10.

**[Table 5]**

| | | Example 12 | Example 13 | Example 14 | Example 15 | Example 16 | Example 17 |
|---|---|---|---|---|---|---|---|
| Microemulsion of Example 3 | | 100 | | | | | |
| Microemulsion of Example 4 | | | 100 | | | | |
| Microemulsion of Example 6 | | | | 100 | | | |
| Microemulsion of Example 8 | | | | | 100 | | |
| Microemulsion of Example 9 | | | | | | 100 | |
| Microemulsion of Example 10 | | | | | | | 100 |
| Solution of chloroplatinic acid-vinylsiloxane complex in toluene | | 0.1 | 0.1 | 0.1 | 0.1 | 0. 1 | 0.1 |
| Appearance of emulsion before reaction | | Colorless, transparent | Colorless , transparent | Translucent | Colorless, transparent | Colorless, transparent | Translucent |
| Appearance of emulsion after reaction | | Colorless , transparent | Colorless , transparent | Translucent | Colorless , transparent | Colorless , transparent | Translucent |
| Pigment solubility test | Water solubility | Homogeneously dissolved | Homogeneously dissolved | Homogeneously dissolved | Homogeneously dissolved | Homogeneously dissolved | Homogeneously dissolved |
| | Oil solubility | Homogeneously dissolved | Homogeneously dissolved | Homogeneously dissolved | Homogeneously dissolved | Homogeneously dissolved | Homogeneously dissolved |
| Water dispersibility | | Homogeneously dispersed | Homogeneously dispersed | Homogeneously dispersed | Homogeneously dispersed | Homogeneously dispersed | Homogeneously dispersed |
| Oil dispersibility | | Separated | Separated | Separated | Separated | Separated | Separated |

As shown in the above Table 5, microemulsion addition-cured compositions having a transparent or translucent appearance at 25°C were achieved in Examples 12 to 17. Since the appearance was thus sustained from before the reaction, it can be conjectured that there was little influence of the curing reaction to phase transition. In addition, in the pigment solubility test, both the water-soluble pigment and the oil-soluble pigment dissolved homogeneously, and therefore, it was confirmed that like the microemulsion before the reaction, the microemulsion addition-cured composition after the reaction had a bicontinuous structure. Furthermore, when the microemulsions were dropped into water, each microemulsion dispersed homogeneously, and thus, dispersibility in water was confirmed. On the other hand, when the microemulsion were dropped into oil, each microemulsion was separated, and was not dispersed in the oil.

5 g of the microemulsion before the reaction and the microemulsion addition-cured composition after the reaction were each dispersed in 95 ml of water, and aqueous dispersions thereof were prepared. About 0.02 g of the prepared aqueous dispersions before and after the reaction were dropped on the back of the hand using a dropper, and spread with the finger to a size of about 2 cm in diameter. After air-drying for 3 minutes, this was rubbed hard with the finger. The microemulsion before the reaction had a stickiness originating from surfactant, but in the microemulsion after the reaction, a solid was twisted and fell off. Thus, progression of the curing reaction was suggested. In addition, a reduction in stickiness by the effect of the solid was observed.

As described, it was confirmed that the inventive microemulsion composition allows, by adding a hydrosilylation catalyst, an addition-curing reaction without losing a transparent appearance, and a transparent or translucent microemulsion addition-cured composition can be produced. In addition, it was confirmed that the inventive microemulsion addition-cured composition has a transparent appearance, and has reduced stickiness originating from an anionic surfactant.

### (Comparative Examples 1 to 4)

In a glass beaker were charged SFNa (KANEKA Surfactin, Kaneka Corporation) as (A) an anionic surfactant, glycerin, being a polyhydric alcohol, as (D) a monohydric or polyhydric alcohol, and a decamethylcyclopentasiloxane (KF-995) or a linear organopolysiloxane (KF-96A-6cs, the organopolysiloxane (7) shown in the following formula, and the organopolysiloxane (8) shown in the following formula) as (G) a silicone oil not containing a hydrosilyl group or an olefinic unsaturated group by the composition shown in Table 6. The mixture was stirred and dissolved at room temperature using a disper, and then (E) water was dropped thereto under room temperature to prepare a microemulsion. The blended amounts are shown by mass%. In addition, Table 6 shows the evaluation of the appearance of the microemulsions at 25°C, and the results of a pigment solubility test, a water dispersibility test, and an oil dispersibility test conducted in the same manner as in Examples 3, 4, 6, and 8 to 10.
<Organopolysiloxane (7)> 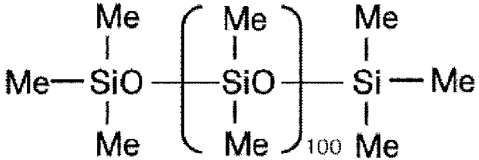
<Organopolysiloxane (8)>

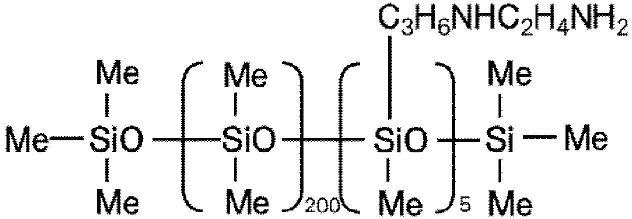

**[Table 6]**

| | | Comparative Example 1 | Comparative Example 2 | Comparative Example 3 | Comparative Example 4 |
|---|---|---|---|---|---|
| Formulated composition (mass %) | SFNa | 1 | 1 | 1 | 1 |
| | Glycerin | 21 | 21 | 21 | 21 |
| | KF-995 | 60 | | | |
| | KF-96A-6cs | | 60 | | |
| | Organopolysiloxane (7) | | | 60 | |
| | Organopolysiloxane (8) | | | | 60 |
| | Water | 18 | 18 | 18 | 18 |
| Total | | 100 | 100 | 100 | 100 |
| Appearance of microemulsion | | Colorless , transparent | Colorless , transparent | Colorless , transparent | Colorless , transparent |
| Pigment solubility test | Water solubility | Homogeneously dissolved | Homogeneously dissolved | Homogeneously dissolved | Homogeneously dissolved |
| | Oil solubility | Homogeneously dissolved | Homogeneously dissolved | Homogeneously dissolved | Homogeneously dissolved |
| Water dispersibility test | | Homogeneously dispersed | Homogeneously dispersed | Homogeneously dispersed | Homogeneously dispersed |
| Oil dispersibility test | | Separated | Separated | Separated | Separated |

As in the above Table 6, microemulsions having a transparent appearance were obtained in Comparative Examples 1 to 4. In addition, in the pigment solubility test, both the water-soluble pigment and the oil-soluble pigment were dissolved homogeneously, and thus, it was confirmed that the obtained microemulsions had a bicontinuous structure. Furthermore, when the microemulsions were dropped into water, each microemulsion dispersed homogeneously, and thus, dispersibility in water was confirmed.

In the same manner as in Examples 12 to 17, about 0.02 g of the prepared aqueous dispersions of Comparative Examples 1 to 4 were dropped on the back of the hand using a dropper, and spread with the finger to a size of about 2 cm in diameter. After air-drying for 3 minutes, this was rubbed hard with the finger. There was stickiness originating from surfactant.

### (Example 18, Comparative Example 5)

### [Property Evaluation]

The feeling on use (absence of stickiness), refreshing feeling (freshness), and temporal stability (state after preserving at 50°C for 1 month) of the prepared cosmetics when cosmetics prepared using the microemulsion addition-cured composition of the Example 12 and the microemulsion of Comparative Example 3 (Example 18, Comparative Example 5) were applied to the skin were evaluated by the evaluation criteria shown in Table 7. The results were judged according to the following judgement criteria on the basis of the average values of experts (10 experts). The results are given together in Table 8. In addition, Example 18 and Comparative Example 5 were prepared according to the prescription shown in Table 8.

### <Preparation of Cosmetics>

By mixing the component (2) in the component (1) in Table 8 uniformly, water-dispersion lotions (cosmetics of Example 18 and Comparative Example 5) were obtained.

**[Table 7]**

| Evaluation criteria | | | |
|---|---|---|---|
| Item | Feeling on use | Refreshing feeling | Temporal stability |
| 5 points | Favorable | Favorable | Favorable |
| 4 points | Somewhat favorable | Somewhat favorable | Somewhat favorable |
| 3 points | Normal | Normal | Normal |
| 2 points | Somewhat unfavorable | Somewhat unfavorable | Somewhat unfavorable |
| 1 point | Unfavorable | Unfavorable | Unfavorable |

### Judgement criteria

Excellent: the average was 4.5 points or more
Good: the average was 3.5 points or more and less than 4.5 points
Fair: the average was 2.5 points or more and less than 3.5 points
Poor: the average was 1.5 points or more and less than 2.5 points
Bad: the average was less than 1.5 points

**[Table 8]**

| Composition (%) | | Example 18 | Comparative Example 5 |
|---|---|---|---|
| (1) | Microemulsion obtained in Example 12 | 5 | - |
| | Microemulsion obtained in Comparative Example 3 | - | 5 |
| (2) | Butyleneglycol | 5 | 5 |
| | Sodium chloride | 0.5 | 0.5 |
| | Preservative | Appropriate amount | Appropriate amount |
| | Purified water | Balance | Balance |
| Total | | 100 | 100 |
| Evaluation | Feeling on use | Excellent | Fair |
| | Refreshing feeling | Excellent | Fair |
| | Temporal stability | Excellent | Fair |

It was revealed from the results of the above Table 8 that the inventive cosmetic (water-dispersion lotion) had favorable feeling on use (absence of stickiness), refreshing feeling (freshness), and temporal stability (state after preserving at 50°C for 1 month) .

In addition, the inventive cosmetic had favorable cosmetic sustainability, favorable smooth spreadability and finish, and excellent abrasion resistance.

It should be noted that the present invention is not limited to the above-described embodiments. The embodiments are just examples, and any examples that have substantially the same feature and demonstrate the same functions and effects as those in the technical concept disclosed in claims of the present invention are included in the technical scope of the present invention.

## Claims

1. A microemulsion composition comprising:
(A) an anionic surfactant;
(B) an organopolysiloxane having at least two hydrosilyl groups in one molecule thereof shown in the following formula (I): 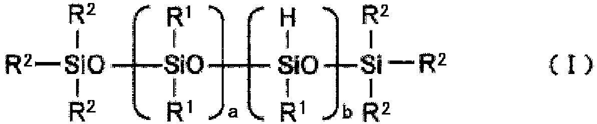 wherein each R¹ independently represents a substituted or unsubstituted monovalent hydrocarbon group having 1 to 30 carbon atoms and not having an aliphatic unsaturated bond, each R² independently represents a substituted or unsubstituted monovalent hydrocarbon group having 1 to 30 carbon atoms and not having an aliphatic unsaturated bond, some optionally being a hydrogen atom, "a" satisfies 0 ≤ a ≤ 300, "b" satisfies 0 ≤ b ≤ 50, and 5 ≤ a + b ≤ 350, and when b = 0, any two or more R² represent a hydrogen atom;
(C) an organopolysiloxane having at least two olefinic unsaturated groups in one molecule thereof shown in the following formula (II): 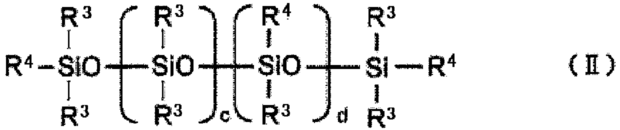 wherein each R³ independently represents a substituted or unsubstituted monovalent hydrocarbon group having 1 to 30 carbon atoms and not having an aliphatic unsaturated bond, each R⁴ independently represents a substituted or unsubstituted monovalent hydrocarbon group having 2 to 30 carbon atoms and having an aliphatic unsaturated bond or is R³, "c" satisfies 0 ≤ c ≤ 500, "d" satisfies 0 ≤ d ≤ 50, and 5 ≤ c + d ≤ 550, and when d = 0, each R⁴ independently represents a substituted or unsubstituted monovalent hydrocarbon group having 2 to 30 carbon atoms and having an aliphatic unsaturated bond;
(D) a monohydric or polyhydric alcohol; and
(E) water,
wherein the microemulsion composition has a transparent or translucent appearance at 25°C.

2. The microemulsion composition according to claim 1, wherein the microemulsion composition is dispersible when added in water.

3. The microemulsion composition according to claim 1 or 2, wherein the (A) anionic surfactant is a natural surfactant.

4. The microemulsion composition according to claim 3, wherein the natural surfactant comprises a cyclic peptide group shown in the following formula (III): 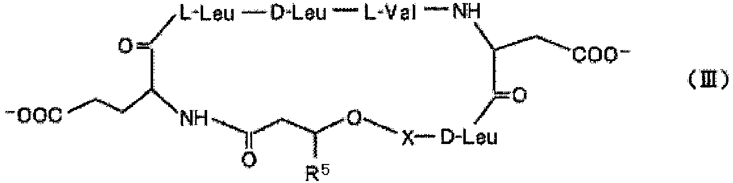 wherein in the formula, X represents an amino acid residue selected from leucine, isoleucine, and valine, each R⁵ independently represents a substituted or unsubstituted monovalent hydrocarbon group having 9 to 18 carbon atoms and not having an aliphatic unsaturated bond, L-Leu indicates L-leucine, D-Leu indicates D-leucine, L-Val indicates L-valine, and a counter ion of a carboxy group comprises an alkali metal ion.

5. The microemulsion composition according to claim 4, wherein in the formula (III), X represents leucine, and R⁵ represents a hydrocarbon chain having 12 carbon atoms.

6. The microemulsion composition according to any one of claims 1 to 5, wherein the (A) anionic surfactant in the microemulsion composition is contained in an amount of 0.1 to 10 wt%.

7. The microemulsion composition according to any one of claims 1 to 6, wherein the (D) monohydric or polyhydric alcohol is glycerin.

8. The microemulsion composition according to any one of claims 1 to 7, wherein the microemulsion composition forms a bicontinuous structure.

9. The microemulsion composition according to any one of claims 1 to 8, wherein there is 0.5 to 3.0 mol of the hydrosilyl groups contained in the (B) relative to 1 mol of the olefinic unsaturated groups contained in the (C).

10. The microemulsion composition according to any one of claims 1 to 9, wherein the microemulsion composition is addition-cured by adding (F) a hydrosilylation catalyst.

11. A microemulsion addition-cured composition obtained by addition-curing the microemulsion composition according to claim 10, wherein the microemulsion addition-cured composition has a transparent or translucent appearance.

12. A cosmetic comprising the microemulsion addition-cured composition according to claim 11.

## Patentansprüche

1. Mikroemulsionszusammensetzung, umfassend:
(A) ein anionisches oberflächenaktives Mittel;
(B) ein Organopolysiloxan, mindestens zwei Hydrosilyl-Gruppen in einem seiner Moleküle aufweisend, dargestellt durch die folgende Formel (I): 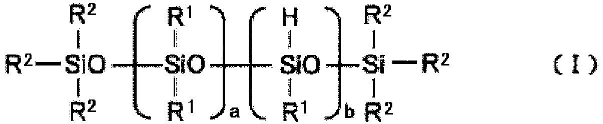 wobei jedes R¹ unabhängig eine substituierte oder unsubstituierte einwertige Kohlenwasserstoffgruppe mit 1 bis 30 Kohlenstoffatomen und ohne aliphatische ungesättigte Bindung darstellt, jedes R² unabhängig eine substituierte oder unsubstituierte einwertige Kohlenwasserstoffgruppe mit 1 bis 30 Kohlenstoffatomen und ohne aliphatische ungesättigte Bindung darstellt, wobei einige optional ein Wasserstoffatom sind, "a" 0 ≤ a ≤ 300 erfüllt, "b" 0 ≤ b ≤ 50 erfüllt, und 5 ≤ a + b ≤ 350, und wenn b = 0, beliebige zwei oder mehr von R² ein Wasserstoffatom darstellen;
(C) ein Organopolysiloxan, mindestens zwei olefinisch ungesättigten Gruppen in einem seiner Moleküle aufweisend, dargestellt durch die folgende Formel (II): 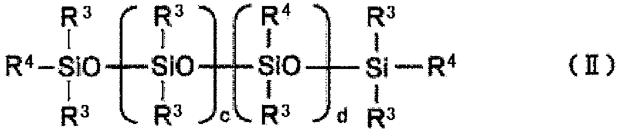 wobei jedes R³ unabhängig eine substituierte oder unsubstituierte einwertige Kohlenwasserstoffgruppe mit 1 bis 30 Kohlenstoffatomen und ohne eine aliphatische ungesättigte Bindung darstellt, jedes R⁴ unabhängig eine substituierte oder unsubstituierte einwertige Kohlenwasserstoffgruppe mit 2 bis 30 Kohlenstoffatomen und mit einer aliphatischen ungesättigten Bindung darstellt oder R³ ist, "c" 0 ≤ c ≤ 500 erfüllt, "d" 0 ≤ d ≤ 50 erfüllt, und 5 ≤ c + d ≤ 550, und wenn d = 0 ist, jedes R⁴ unabhängig eine substituierte oder unsubstituierte einwertige Kohlenwasserstoffgruppe mit 2 bis 30 Kohlenstoffatomen und mit einer aliphatischen ungesättigten Bindung darstellt;
(D) einen einwertigen oder mehrwertigen Alkohol; und
(E) Wasser,
wobei die Mikroemulsionszusammensetzung bei 25°C ein transparentes oder transluzentes Erscheinungsbild aufweist.

2. Die Mikroemulsionszusammensetzung nach Anspruch 1, wobei die Mikroemulsionszusammensetzung bei Zugabe in Wasser dispergierbar ist.

3. Die Mikroemulsionszusammensetzung nach Anspruch 1 oder 2, wobei das (A) anionische oberflächenaktive Mittel ein natürliches oberflächenaktives Mittel ist.

4. Die Mikroemulsionszusammensetzung nach Anspruch 3, wobei das natürliche oberflächenaktive Mittel eine cyclische Peptidgruppe, dargestellt durch die folgende Formel (III), umfasst: 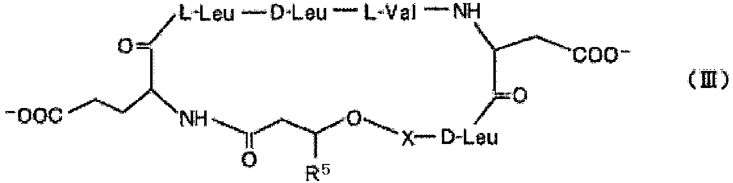 wobei in der Formel X einen Aminosäurerest, ausgewählt aus Leucin, Isoleucin und Valin, darstellt, jedes R⁵ unabhängig eine substituierte oder unsubstituierte einwertige Kohlenwasserstoffgruppe mit 9 bis 18 Kohlenstoffatomen und ohne eine aliphatische ungesättigte Bindung darstellt, L-Leu gleich L-Leucin, D-Leu gleich D-Leucin, L-Val gleich L-Valin bedeutet, und ein Gegenion einer Carboxygruppe ein Alkalimetallion umfasst.

5. Die Mikroemulsionszusammensetzung nach Anspruch 4, wobei in der Formel (III) X gleich Leucin darstellt und R⁵ eine Kohlenwasserstoffkette mit 12 Kohlenstoffatomen darstellt.

6. Die Mikroemulsionszusammensetzung nach einem der Ansprüche 1 bis 5, wobei das (A) anionische oberflächenaktive Mittel in der Mikroemulsionszusammensetzung in einer Menge von 0,1 bis 10 Gew.-% enthalten ist.

7. Die Mikroemulsionszusammensetzung nach einem der Ansprüche 1 bis 6, wobei der (D) einwertige oder mehrwertige Alkohol Glycerin ist.

8. Die Mikroemulsionszusammensetzung nach einem der Ansprüche 1 bis 7, wobei die Mikroemulsionszusammensetzung eine bikontinuierliche Struktur bildet.

9. Die Mikroemulsionszusammensetzung nach einem der Ansprüche 1 bis 8, wobei 0,5 bis 3,0 mol der in (B) enthaltenen Hydrosilylgruppen bezogen auf 1 mol der in (C) enthaltenen olefinisch ungesättigten Gruppen vorhanden sind.

10. Die Mikroemulsionszusammensetzung nach einem der Ansprüche 1 bis 9, wobei die Mikroemulsionszusammensetzung durch Zugabe (F) eines Hydrosilylierungskatalysators additionsgehärtet ist.

11. Additionsgehärtete Mikroemulsionszusammensetzung, erhalten durch Additionshärtung der Mikroemulsionszusammensetzung nach Anspruch 10, wobei die additionsgehärtete Mikroemulsionszusammensetzung ein transparentes oder transluzentes Erscheinungsbild aufweist.

12. Kosmetikum, umfassend die additionsgehärtete Mikroemulsionszusammensetzung nach Anspruch 11.

## Revendications

1. Composition de microémulsion comprenant :
(A) un tensioactif anionique ;
(B) un organopolysiloxane ayant au moins deux groupes hydrosilyle dans une molécule de celui-ci représenté dans la formule (I) suivante : 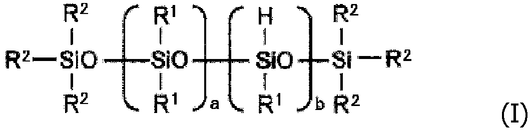 dans laquelle chaque R¹ représente indépendamment un groupe hydrocarboné monovalent substitué ou non substitué ayant de 1 à 30 atomes de carbone et n'ayant pas de liaison aliphatique insaturée, chaque R² représente indépendamment un groupe hydrocarboné monovalent substitué ou non substitué ayant de 1 à 30 atomes de carbone et n'ayant pas de liaison aliphatique insaturée, certains étant éventuellement un atome d'hydrogène, « a » satisfait 0 ≤ a ≤ 300, « b » satisfait 0 ≤ b ≤ 50, et 5 ≤ a+ b ≤ 350, et lorsque b = 0, deux R² quelconques ou plus représentent un atome d'hydrogène ;
(C) un organopolysiloxane ayant au moins deux groupes oléfiniques insaturés dans une molécule de celui-ci représenté dans la formule (II) suivante : 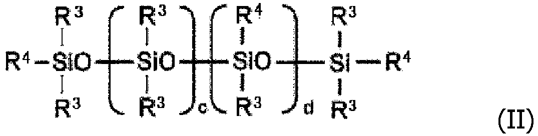 dans laquelle chaque R³ représente indépendamment un groupe hydrocarboné monovalent substitué ou non substitué ayant de 1 à 30 atomes de carbone et n'ayant pas de liaison aliphatique insaturée, chaque R⁴ représente indépendamment un groupe hydrocarboné monovalent substitué ou non substitué ayant de 2 à 30 atomes de carbone et ayant une liaison aliphatique insaturée ou est R³, « c » satisfait 0 ≤ c ≤ 500, « d » satisfait 0 ≤ d ≤ 50, et 5 ≤ c + d ≤ 550, et lorsque d = 0, chaque R⁴ représente indépendamment un groupe hydrocarboné monovalent substitué ou non substitué ayant de 2 à 30 atomes de carbone et ayant une liaison aliphatique insaturée ;
(D) un alcool monohydrique ou polyhydrique ; et
(E) de l'eau,
dans laquelle la composition de microémulsion a un aspect transparent ou translucide à 25 °C.

2. Composition de microémulsion selon la revendication 1, dans laquelle la composition de microémulsion est dispersible lorsqu'elle est ajoutée à de l'eau.

3. Composition de microémulsion selon la revendication 1 ou 2, dans laquelle le tensioactif anionique (A) est un tensioactif naturel.

4. Composition de microémulsion selon la revendication 3, dans laquelle le tensioactif naturel comprend un groupement peptidique cyclique représenté dans la formule (III) suivante : 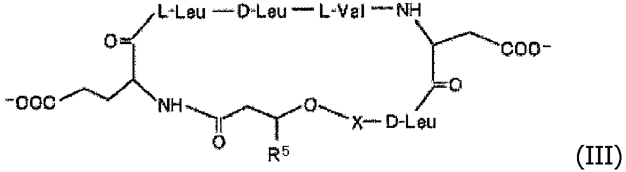 dans laquelle dans la formule, X représente un résidu d'acide aminé choisi parmi la leucine, l'isoleucine et la valine, chaque R⁵ représente indépendamment un groupement hydrocarboné monovalent substitué ou non substitué ayant de 9 à 18 atomes de carbone et n'ayant pas de liaison aliphatique insaturée, L-Leu indique la L-leucine, D-Leu indique la D-leucine, L-Val indique la L-valine, et un contre-ion d'un groupe carboxy comprend un ion de métal alcalin.

5. Composition de microémulsion selon la revendication 4, dans laquelle dans la formule (III), X représente la leucine, et R⁵ représente une chaîne hydrocarbonée ayant 12 atomes de carbone.

6. Composition de microémulsion selon l'une quelconque des revendications 1 à 5, dans laquelle le tensioactif anionique (A) dans la composition de microémulsion est contenu en une quantité de 0,1 à 10 % en poids.

7. Composition de microémulsion selon l'une quelconque des revendications 1 à 6, dans laquelle l'alcool monohydrique ou polyhydrique (D) est la glycérine.

8. Composition de microémulsion selon l'une quelconque des revendications 1 à 7, dans laquelle la composition de microémulsion forme une structure bicontinue.

9. Composition de microémulsion selon l'une quelconque des revendications 1 à 8, dans laquelle il y a 0,5 à 3,0 moles des groupes hydrosilyle contenus dans le (B) par rapport à 1 mole des groupes oléfiniques insaturés contenus dans le (C).

10. Composition de microémulsion selon l'une quelconque des revendications 1 à 9, dans laquelle la composition de microémulsion est polymérisée par addition en ajoutant un catalyseur d'hydrosilylation (F).

11. Composition de microémulsion polymérisée par addition obtenue par polymérisation par addition de la composition de microémulsion selon la revendication 10, dans laquelle la composition de microémulsion polymérisée par addition a un aspect transparent ou translucide.

12. Produit cosmétique comprenant la composition de microémulsion polymérisée par addition selon la revendication 11.
